(19) Europäisches Patentamt
European Patent Office
Office européen des brevets

(11) **EP 4 142 853 B1**

(12) **EUROPÄISCHE PATENTSCHRIFT**

(45) Veröffentlichungstag und Bekanntmachung des Hinweises auf die Patenterteilung:
**20.03.2024 Patentblatt 2024/12**

(21) Anmeldenummer: **21727549.4**

(22) Anmeldetag: **28.04.2021**

(51) Internationale Patentklassifikation (IPC):
**A61M 25/10** (2013.01)   **A61M 31/00** (2006.01)
**A61F 5/44** (2006.01)   **A61F 5/451** (2006.01)

(52) Gemeinsame Patentklassifikation (CPC):
(C-Sets verfügbar)
**A61L 29/041; A61L 29/06;** A61F 5/44;
A61M 25/1002; A61M 2202/068; A61M 2205/0238;
A61M 2210/1064; A61M 2210/1067   (Forts.)

(86) Internationale Anmeldenummer:
**PCT/IB2021/053556**

(87) Internationale Veröffentlichungsnummer:
**WO 2021/220207 (04.11.2021 Gazette 2021/44)**

(54) **GESCHLOSSENES SYSTEM FÜR DIE MÖGLICHST GERUCHSNEUTRALE ZU- UND/ODER ABLEITUNG VON GERUCHSINTENSIVEN SUBSTANZEN ZU/VON DEM KÖRPER EINES PATIENTEN**

CLOSED SYSTEM FOR AS FAR AS POSSIBLE ODOUR-NEUTRAL DELIVERY AND/OR DISCHARGE OF ODOUR-INTENSIVE SUBSTANCES TO/FROM THE BODY OF A PATIENT

SYSTÈME FERMÉ POUR UNE ADMINISTRATION ET/OU UNE ÉVACUATION AU MAXIMUM NEUTRE EN ODEURS DE SUBSTANCES ODORANTES DANS/DEPUIS LE CORPS D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **28.04.2020 DE 102020002764**
**18.05.2020 PCT/IB2020/054684**

(43) Veröffentlichungstag der Anmeldung:
**08.03.2023 Patentblatt 2023/10**

(73) Patentinhaber: **Advanced Medical Balloons GmbH**
**68753 Waghäusel (DE)**

(72) Erfinder: **GÖBEL, Fred**
**67346 Speyer (DE)**

(74) Vertreter: **Küchler, Stefan**
**Stefan T. Küchler**
**Patentanwalt**
**Färberstraße 20**
**90402 Nürnberg (DE)**

(56) Entgegenhaltungen:
**EP-A2- 0 625 343**   **EP-A2- 1 514 528**
**WO-A1-2011/142928**   **WO-A1-2013/056013**
**WO-A2-2008/052018**   **WO-A2-2011/138731**
**US-A1- 2011 125 114**   **US-A1- 2015 282 978**

(52) Gemeinsame Patentklassifikation (CPC): (Forts.)

C-Sets
**A61L 29/041, C08L 27/06;**
**A61L 29/041, C08L 29/04;**
**A61L 29/06, C08L 75/04;**
**A61L 29/06, C08L 77/00;**
A61M 2202/068, A61M 2202/0014

**Beschreibung**

Die Erfindung richtet sich auf ein passager oder auch kontinuierlich im Körper eines Patienten verweilendes, geschlossenes System für die Zu- und/oder Ableitung von geruchsintensiven Substanzen, wobei die Freisetzung von Geruchsstoffen in die Umgebung des Patienten auf ein kleinst mögliches Maß reduziert werden soll, und wobei ggf. eine geruchsneutrale Dekompression von im Körper entstehenden und in das System gelangenden, gasförmigen Anteilen gewährleistet sein soll.

Das System umfasst eine zu- und/oder ableitende, katheterartige Struktur zu einem Organ oder einem sonstigen Binnenraum des Körpers, welche mit einer, der katheterartigen Struktur endständig aufsitzenden, ballonartigen Komponente zur Verankerung und/oder Dichtung des Systems innerhalb des jeweiligen Körperhohlraums ausgestattet ist, sowie einen extrakorporal angeordneten Behälter für die Aufnahme der jeweiligen zu- und/oder abzuleitenden Substanz, sowie eine schlauch- oder schlauchfolienartige Leitung, die das Lumen der im Körper positionierten, katheterartigen Struktur mit dem extrakorporalen Behälter verbindet.

Katheter für die kontinuierliche Ableitung und Sammlung von Stuhl aus dem Rektum eines Patienten sind etablierter Bestandteil der intensivmedizinischen Versorgung. Stuhldrainagen konventioneller Bauart bestehen aus einem torusförmigen Ballon, der den Katheter im Rektum bzw. auf dem Boden des Rektums aufliegend verankert, sowie einem, die Ballonkomponente tragenden, durch den analen Schließmuskel hindurchreichenden Schlauchelement, welches in Verlängerung an einen extrakorporal angeordneten Sammelbehälter konnektiert.

Stuhlableitende Drainagevorrichtungen der beschriebenen einfachen Bauart sind konzeptionell nicht in der Lage dünnflüssige Stühle sicher geschlossen abzuleiten. Eine Verschmutzung der perianalen Hautpartien sowie eine Kontamination der unmittelbaren Pflegeumgebung des Patienten kann nicht verhindert werden, Die Problematik der Dichtung des Schließmuskels bleibt im Design dieser Vorrichtungen vollständig unberücksichtigt. Das durch den Analkanal reichende, stuhlableitende Schlauchsegment geht bei normalen Tonus, des dem Schlauchsegment anliegenden Schließmuskels, in eine typische, einfache oder mehrfache, radiale Faltung über, wobei das Schlauchsegment grobe, längsverlaufende Kanäle formiert, die flüssigen Darminhalt aus dem Rektum in den prä-analen Bereich leiten. Um die so forcierte Leckage von Stuhl zu begrenzen, wurde bei einigen Ausführungen dieses einfachen Bautyps ein besonders dünnwandiges und weiches, transanales Schlauchsegment integriert. Die entsprechend ausgestatteten Segmente begünstigen jedoch okkludierende Verwindungen des stuhlableitenden Lumens der Vorrichtung.

Konventionelle Drainagesysteme für die kontinuierliche Ableitung von Stuhl bestehen in aller Regel sowohl im intrakorporalen, rektal stuhlaufnehmenden und trans-anal ableitenden Kopfteil, als auch im extrakorporalen, zum Sammelbehälter führenden Schlauchteil, vollständig aus Silikon.

Silikone sind insbesondere bei der Verarbeitung zu dünnwandigen Ballon- und Schlauchstrukturen nur sehr unzureichend in der Lage, die Freisetzung von geruchsaktiven Substanzen zu verhindern oder auf ein tolerables Maß zu reduzieren. Werden native, unbeschichtete, oder sonstig unbehandelte silikonbasierte Komponenten flüssigem Exkrement exponiert, kann sich bereits nach wenigen Stunden an den zur Umgebung hin gerichteten Oberflächen ein äußerst intensiver Geruch entwickeln. Der vom System ausgehende Geruch kann von Patienten und Anwendern als derart belastend empfunden werden, dass die kontinuierliche Stuhlableitung abgebrochen werden muss.

Hersteller statten silikonbasierte Stuhlableitungssysteme daher im Bereich des extrakorporalen, zum Sammelbehälter führenden Schlauchsegmentes häufig mit barriere-wirksamen, den Durchtritt von Geruchsstoffen hemmenden, Geruchsstoffe adsorbierenden, oder Geruchsstoffe neutralisierenden Hilfsstoffen aus. Bedingt durch die in der Regel ausgeprägte Steifigkeit der entsprechenden Materialien, wie beispielsweise Parylene, welches als Beschichtung z.B. durch ein Tauchverfahren aufgetragen wird, werden die intrakorporalen und anusnahen Schlauchanteile typischerweise nicht mit derartigen geruchsreduzierenden Materiallagen versehen.

Silikon-basierte Kathetervorrichtungen lassen keine mehrlagige Kombination mit gängigen Barrierematerialien, wie beispielsweise EVOH oder PVDC zu. Beschichtungen von Silikonkathetem erfordern in der Regel vorausgehende, oberflächenaktivierende Verfahrensschritte, wie z.B. die Aktivierung mit Plasma.

Die Entgegenhaltung WO2011/142928 A1 offenbart eine Kombination aus einem Fäkaldrainagekatheter mit einem distalen Teil zum Einführen in das Rektum und einem Drainagerohr zum Ableiten von Fäkalien aus dem Rektum, und einem oder mehreren Materialien zur Freisetzung von Chlordioxidgas, wenn es aktiviert wird, wobei das Chlordioxidgas entlang des Drainagerohrs zur Geruchs- und Bakterienkontrolle durchdringt.

Die Entgegenhaltung WO2011/138731 A2 ist auf eine Einrichtung zum Anschluss an einen künstlichen Darmausgang gerichtet, mit einer in situ intrakoporal platzierbaren Kopfeinheit mit einem Ballon, sowie einer extrakorporalen Beuteleinheit.

Die Entgegenhaltungen US 2015/0282978 A1, WO2013/056013 A1, US2011/0125114 A1 und EP0625343A2 betreffen neutrale Materialien. US 2015/0282978 A1 ist auf eine geruchsdichte Folie aus mehreren Lagen gerichtet. WO 2013/056013 A1 offenbart einen Polymerfilm. US2011/0125114 A1 beschreibt eine schalldämpfende Folie. EP0625343 A2 spezifiziert eine Geruchsbarriere für ein medizinisches Gerät spezifiziert.

Eine weitere Entgegenhaltung EP1514528 A2 ist auf einen Filter für einen Beutel gerichtet.

Ferner offenbart WO2008/052018 A2 eine rektal einführbare Kopfeinheit mit einem Ballon und mit einer Röhre zum Abtransport übelriechender Materialien aus dem Körper eines Menschen.

Seit einigen Jahren sind im Markt neuartige Stuhlableitungssysteme mit besonderen, trans-anal positionierten und dichtenden Kopfeinheiten aus Polyurethan (PUR) bekannt, wie beispielsweise der Typ hyghtec® basic-plus der Firma Creative Ballons GmbH, Waghäusel, Deutschland. Dieser Drainagetyp weist einen, sich an die Kopfeinheit anschließenden, optional aus PUR oder PVC hergestellten, extrakorporalen Ableitungsschlauch auf. Sowohl PUR als auch PVC, als auch mögliche Compounds oder Koextrusionen der beiden Materialien, erweisen sich bei längeren Verweilzeiten im Körper ebenfalls nur unzureichend geruchsdicht.

Während die trans-anal positionierte Kopfeinheit dieses neuartigen Bautyps nahezu vollständig innerhalb des Körpers des Patienten liegt, und lediglich ein kleines, sphärisch erweitertes Segment des trans-analen Ballonkorpus aus dem Anus herausragt, entwickelt die zum Sammelbeutel führende, stuhlableitende Schlaucheinheil eine deutlich größere, geruchswirksame Gesamtfläche von ca. 1400 cm$^2$, ausgehend von einem Schlauchdurchmesser von 2,5 cm und einer Schlauchlänge von 180 cm.

Aus den Nachteilen des beschriebenen Standes der Technik resultiert das die Erfindung initiierende Problem, ein gattungsgemäßes System derart weiterzubilden, dass die Geruchsfreisetzung bei der kontinuierlichen Ableitung von Stuhl oder auch von anderen geruchsintensiven Exkreten, Sekreten oder sonstigen Körperflüssigkeiten, sowie bei der Zuleitung von geruchsintensiven Substanzen in den menschlichen Körper, als auch bei der Dekompression von gasartigen Anteilen, die aus dem Körper in das zu- und/oder ableitende System gelangen, minimiert oder weitestgehend vermieden wird.

Die Lösung dieses Problems gelingt dadurch, dass zumindest die extrakorporalen Bestandteile der zu- und/oder ableitenden Vorrichtung, umfassend den, die jeweilige Substanz aufnehmenden Behälter, und die, den intrakorporalen Teil der Vorrichtung mit dem Behälter verbindende Schlauchleitung, jeweils aus einem mehrlagigen Folienmaterial bestehen, das jeweils wenigstens eine Lage eines effizient barrierewirksamen Materials, wie z.B. Ethylen-Vinylalkohol-Copolymer (EVOH) und/oder Polyvinylidenchlorid (PVDC) integriert, wobei das jeweilige Barrierematerial mit einer, zwei- oder mehreren Lagen robuster, mechanisch belastbarer Tragerfolie kombiniert sind, und wobei distal zum, die jeweilige Substanz aufnehmenden Kompartiment des Behälters eine Entgasungseinheit vorgesehen ist, über die gasförmige Anteile aus der Vorrichtung, einem Druckgefälle folgend, in die Umgebung abgeleitet werden, und wobei die Entgasungseinheit eine geruchsbindende oder sonstig neutralisierende Adsorptions- und/oder Filtereinrichtung integriert oder jener nachgeordnet ist.

Die Erfindung wird in dem unabhängigen Anspruch 1 definiert. Die abhängigen Ansprüche sind auf die bevorzugten Ausführungsformen der Erfindung gerichtet.

Die vorliegende Erfindung beschreibt insbesondere technische Lösungswege zur Eindämmung bzw, weitestgehenden Vermeidung einer Geruchsfreisetzung bei der kontinuierlichen Ableitung bzw. Drainage von Stuhl oder auch von anderen geruchsintensiven Exkreten, Sekreten oder sonstigen Körperflüssigkeiten. Die Erfindung umfasst ferner die geruchsreduzierende bzw. geruchsvermeidende Zuleitung geruchsintensiver, beispielsweise therapeutische Substanzen zum Körper.

Die Erfindung bietet vorzugsweise geruchsreduzierende, technische Lösungen für die extrakorporale Einheit des Systems, bestehend aus dem Verbund von Zuleitungschlauch und Behälter, bezieht konzeptionell aber auch die stuhlexponierten und potenziell geruchsfreisetzenden Oberflächen der im Körper verweilienden, intrakorporalen Einheit des Systems anteilig oder vollständig mit ein.

Die beschriebene Schlauch- und Folientechnologie beruht auf der Verwendung mehrlagiger Materialkombinationen, wobei eine oder mehrere Lagen eines beständigen, mechanisch robusten Trägermaterials, mit wenigstens einer Lage eines geruchsdichten . Barrierematerials, zu einer mehrlagigen Gesamtfolienstruktur verbunden werden, beispielsweise durch einen Koextrusionsprozess, einen Laminierungsprozess oder auch mehrschrittigen Tauchprozess.

Die im Rahmen der vorliegenden Erfindung vorgestellten, mehrlagig aufgebauten Schlauchleitungen für die Zu- und/oder Ableitung von Substanzen umfassen eine oder mehrere Lagen eines mechanisch belastbaren, bevorzugt elastisch verformbaren und sich elastisch aufrichtenden Trägermaterials, wobei bevorzugt thermoplastische Polyurethane (TPU) in Shore-Härtebereich von 80A bis 95A verwendet werden. Weniger bevorzugt können auch Polyvinylchlorid (PVC), Polyamid (PA), ehermoplastische Polyamidelastomere (TPE-A) oder auch beispielsweise polyolefin-basierte Polymere, wie z.B. Polyethylen niedriger Dichte (LDPE) zur Anwendung kommen. Weniger geeignet ist Silikon, da weder EVOH noch PVDC aufgrund ihrer chemischen Eigenschaften mit Silikon kombinierbar, insbesondere nicht als mehrlagig koextrudierte, mehrlagig laminierte oder getauchte Rohlinge herstellbar sind.

Das jeweilige Schlauchmaterial kann neben ' einer sandwich-artigen Anordnung einer mittig positionierten Lage aus EVOH oder PVCD zwischen zwei, den Schlauch stabilisierenden Trägerlagen, optional auch aus einem zwei-lagigen, nur eine Trägerlage umfassenden Materialverbund aufgebaut sein. Während sich die Barriere-Eigenschaften von EVOH bei direkter Exposition mit wässrigen Substanzen verschlechtern, kann PVDC auch in einem wässrigen Milieu ohne Verlust seiner Barrierewirkung verwendet werden.

EVOH und PVDC weisen auch in relativ niedrigen Wandungsstärken eine relativ ausgeprägte Steifigkeit auf.

Um dennoch eine körperfreundliche . Weichfoliencharakteristik einer mehrlagigen, mit EVOH oder PVDC ausgestatteten Folie zu erreichen, wird die Stäre der Barrierelage relativ zu den jeweiligen Trägerlagen möglichst dünnwandig ausgeführt. Die Summe der Lagenstärken der Barrierematerialien liegt beispielsweise im Bereich eines Zehntels bis Zwanzigstels oder auch eines Zwanzigstels bis Dreissigstels der Summe der Lagenstärken der Trägermaterialien.

Das mehrlagige, extrakorporale Schlauchsegment wird bevorzugt derartig dünnwandig ausgeführt, dass es bereits bei kleiner Krafteinwirkung von außen zu einer flachen, bandartigen Strukturen kollabiert, und somit der Entstehung von druckbedingten Läsionen der Haut des Patienten vorbeugt, wenn beispielsweise der Körperstamm oder die Extremitäten des Patienten dem Schlauchsegment passager aufliegen. In der bevorzugten Ausführung des Schlauches richtet sich dieser, bei nachlassender äußerer Krafteinwirkung, zumindest partiell, spontan elastisch in sein bei der Herstellung ausgeformtes Ursprungsprofil auf. Ein entsprechendes elastisches Aufrichtungsverhalten kann insbesondere durch die Verwendung von PUR als kombinierendes Trägermaterial realisiert werden. Zur Verwendung kommen beispielsweise PUR Typen der Shore Härte 80A bis 95A oder auch 55D bis 65D. Der Durchmesser derartig elastisch wirkender, mit einer PUR-basierten Trägerlage ausgerüsteter, zu- und/oder ableitender Schlauchsegmente liegt beispielsweise bei stuhlableitenden Systemen im Bereich von 15 bis 30 mm, bevorzugt 20 bis 25 mm. Die Wandungsstärke der Schlauchkomponente liegt typischerweise im Bereich von 200 bis 500 $\mu$m. Die Stärke der Barrierelage liegt bei etwa 5 bis 25 $\mu$m, bevorzugt 10 bis 15 $\mu$m.

Die spontane Aufrichtung bzw. Rundung des Querschnitts nach einer passageren Verformung des extrakorporalen, stuhlableitenden Schlauchsegments durch eine von außen auf den Schlauch wirkende Kraft kann durch seriell aufeinanderfolgend angeordnete, ringförmige, konvex oder konkav gerichtete Ausstülpungen oder Einstülpungen der Schlauchwandung unterstützt werden. Bei den zuvor genannten Materialhärten, Wandungsstärken und Schlauchdurchmessern sind solche zirkulären Erweiterungen oder Reduktionen des Schlauchmantels bevorzugt 3 bis 6 mm breit und weisen Auslenkungen des Schlauchdurchmessers von im Scheitel 1,0 bis 3,0 mm, bevorzugt 1,5 bis 2,0 mm auf. Die Auslenkungen sind in der bevorzugten Ausführung jeweils bogenförmig, konvex nach außen oder konkav nach innen gewölbt.

Bei alleiniger Verwendung eines PVC-basierten Trägermaterials in Kombination mit einer EVOH oder PVDC basierten Trennschicht, sind die im Rahmen der Erfindung bevorzugten, PUR-typischen, elastischen Aufrichtungseigenschaften des Schlauchmantels nicht oder nur in kleinem Umfang erreichbar. Bei einer anteiligen Verwendung von PVC, als beispielsweise Innen- oder Außenlage, kann die Fähigkeit zur elastischen Selbstaufrichtung jedoch durch Aufnahme einer zusätzlichen PUR Lage in die Folienwandung integriert werden. Das anteilige PUR Lage weist dann vorzugsweise eine höhere Shore-Härte auf, die besipielsweise im Bereich von Shore 55D bis 65D liegt. Bei einer angestrebten Gesamtwandungsstärke der Schlauchfolienwandung für einen stuhlableitenden Drainageschlauch, von 200 bis 400 $\mu$m, kann beispielhaft folgende Anordnung von Materiallagen kombiniert werden: außen - PUR 55D (50-100$\mu$m), mittig - EVOH oder PVDC (10-20$\mu$m), innen - PVC 60A-80A (140 bis 280$\mu$m). Eine nach innen, zum Drainagelumen ausgerichtete PVC-Lage ist im Rahmen der Erfindung konzeptionell von Vorteil, da der mit PVC erreichbare Barriereeffekt gegen Wasser den entsprechenden Barriereeffekt einer PUR-Lage gleicher Wandungsstärke übersteigt. Bei der Verwendung von EVOH als mittig verbaute Barriere-Lage ist ein Schutz vor Wassermolekülen von Vorteil, da die Barriere-Wirkung von EVOH durch eine Exposition mit Wasser reduziert wird.

PUR-basierte Materiallagen statten insbesondere die im Rahmen der Erfindung beschriebenen, mehrlagig aufgebauten, aus Schlauchfolienmaterial hergestellten Komponenten der Kathetervorrichtung mit einer hohen mechanischen Stabilität und Belastbarkeit aus. Bereits dünnwandige, anteilige PUR-Lagen im Bereich von 10 bis 30 $\mu$m verleihen der jeweiligen Komponente, im Vergleich zu beispielsweise PVC, eine erheblich verbesserte Zug- und Reißfestigkeit, sowie Schnitt- und Punktionsresistenz.

Die Erfindung beschreibt neben Schlauchfolien auch Flachfolienmaterial, das einen, dem beschriebenen Schlauchmaterial entsprechenden, mehrlagigen, eine oder mehrere Barrierelagen integrierenden Wandungsaufbau aufweist. Die Flachfolien werden erfindungsgemäßen System für Herstellung des die jeweilige zu- und/oder ableitende Substanz aufnehmenden Gefäßes bzw. Beutels verwendet. Die Flachfolien können optional allerdings auch zu zu- und/oder ableitendem Schlauchfolienmaterial weiterverarbeitet werden.

Die erfindungsgemäß eingestzten Trägermateriallen PUR und PVC, sind mit gängigen Lösungsmitteln, wie beispielsweise Cyclohexanon oder Tetrahydrofuran verklebbar, was für die einfache Montage einer Kathetervorrichtung aus mehreren, separat hergestellten Komponenten und Baugruppen entscheidend ist. In den bevorzugten Ausführungen der hier beschriebenen, geruchsdicht optimierten Kathetervorrichtung, wird das stuhlableitende Schlauchsegment zwischen einer distalen, im Rektum platzierten, vorzugsweuse PUR-basierten Kopfeinheit und einem proximalen, extrakorporalen Konnektorelement aus beispielsweise PVC oder ABS, jeweils durch Lösungsmittelklebungen verbunden.

Neben EVOH oder PVDC können, als weniger wirksame und entsprechend weniger bevorzugte Geruchsbarriere auch anteilige Lagen von Polyamid (PA) oder thermoplastischem Polyamidelastomer (TPE-A) in eine erfindungsgemäße, mehrlagige Schlauch- oder Folie verbaut werden.

Vorteilhaft sind u.a. Lagenkombinationen aus EVOH und PA, da sich beide Materialien insbesondere bei Koextrusionsverfahren gut und in der Regel ohne einen, die Haftung unterstützenden Tie-Layer-Lage verbinden lassen.

Ferner bieten sich Lagenkombinationen aus PA und PUR an, die ebenfalls die Option auf Koextrusion ohne haftungsvermittelnden Tie-Layer bietet. Durch Einsatz entsprechender Haftungsvermittler als Zwischenlage sind wiederum Verbindungen von PA oder PUR mit beispielsweise LDPE-basierten Lagen möglich.

Die Erfindung schlägt u.a. mehrlagige Folien vor, die auf eine EVOH- oder PVDC-basierte Barrierelage verzichten, und stattdessen eine oder mehrere Polyamidlagen mit einer oder mehreren PUR-Trägerlagen kombinieren. Die geruchsreduzierenden Barriereeigenschaften von PA erreichen nicht die Effizienz von EVOH oder PVDC, sind jedoch bei kürzeren Anwendungszeiten einer erfindungsgemäßen Kathetervorrichtung vorteilhaft nutzbar.

Die für die Herstellung der intrakorporal und extrakorporal verbauten, ballon- und schlauchfolienartigen Komponenten eingesetzten, mehrlagig hergestellten Schlauchrohlinge, werden durch einen nachfolgenden Herstellungsschritt, beispielsweise durch ein hot-molding Verfahren, thermisch umgeformt, wobei der unbearbeitete Schlauchrohling, durch Beaufschlagung mit Druckluft, in eine erhitze Formkavität expandiert, und dort durch anschließende Kühlung des Formwerkzeuges in seiner Form fixiert wird. Neben einer derartigen Umformung des extra-korporalen, stuhlableitenden Schlauchsegmentes zu einem beispielsweise mit lumen-stabilisierenden Erweiterungen oder Einziehungen versehenen Schlauchmantel, umfasst die Erfindung auch Ausführungsformen, bei denen neben der Ausformung des stuhlableitenden Schlauchsegmentes eine gleichzeitige Ausformung der intra-korporalen Schaftschlauchkomponente sowie, optional auch der intra-korporalen Ballonkomponente der Vorrichtung erfolgt. Die Erfindung beschreibt besondere Ausführungsformen der Kathetervorrichtung, bei denen sowohl der gesamte intra- als auch der gesamte extra-korporale Anteil des Katheters aus einem einzigen eingesetzten, strukturell durchgängigen Folienschlauchrohling ausgeformt wird.

Weitere Merkmale, Einzelheiten, Vorteile und Wirkungen auf der Basis der Erfindung ergeben sich aus der folgenden Beschreibung einer bevorzugten Ausführungsform der Erfindung sowie anhand der Zeichnung. Hierbei zeigt:

Fig. 1    eine beispielhafte Kathetervorrichtung, umfassend eine aufnehmende Kopfeinheit, eine ableitende Schlaucheinheit, sowie eine an die Schlaucheinheit konnektierte Sammeleinheit,

Fig. 2    eine andere Ausführungsform einer Kopfeinheit für ein erfindungsgemäßes System,

Fig. 3    einen Schnitt durch die Fig. 2 entlang der Linie III - III,

Fig. 4    eine wiederum andere Ausführungsform einer Kopfeinheit für ein erfindungsgemäßes System,

Fig. 5    eine nochmals abgewandelte Ausführungsform einer Kopfeinheit für ein erfindungsgemäßes System,

Fig. 6    eine weiter veränderte Ausführungsform einer Kopfeinheit für ein erfindungsgemäßes System,

Fig. 7    eine abweichende Ausführungsform einer Kopfeinheit für ein erfindungsgemäßes System;

Fig. 8    eine wiederum veränderte Ausführungsform einer Kopfeinheit für ein erfindungsgemäßes System,

Fig. 9    eine unterschiedliche Ausführungsform einer Kopfeinheit für ein erfindungsgemäßes System;

Fig. 10   eine noch andere Ausführungsform einer Kopfeinheit für ein erfindungsgemäßes System,

Fig. 11   einen Schnitt durch die Fig. 10 entlang der Linie XI - XI,

Fig. 12   eine nochmals abgewandelte Ausführungsform einer Kopfeinheit für ein erfindungsgemäßes System,

Fig. 13   eine weiter abgeänderte Ausführungsform einer Kopfeinheit für ein erfindungsgemäßes System,

Fig. 14   einen Behälter für ein erfindungsgemäßes System,

Fig. 15   die Verbindung zwischen einem Behälter und einer jenem vorgeschalteten Entgasungs-Einrichtung,

Fig. 16   eine Entgasungs-Einrichtung in einer lateralen, seitlichen Anordnung zu einem drainierenden oder stuhlableitenden Grundelement der Vorrichtung,

Fig. 17   eine andere Ausführungsform einer Entgasungs-Einrichung nach Art eines T-Stücks, wobei die dekomprimierende und/oder filternde Einrichtung reversibel lösbar oder auch fix auf dem freien Ende des seitlich abgehenden Schenkels aufgesteckt bzw. auf der Außenwandung des Schenkels konnektiert ist,

Fig. 18    eine weitere Ausführungsform einer erfindungsgemäßen Entgasungs-Einrichtung nach Art eines T-Stücks, wobei die jeweilige dekomprimierende und/oder filternde Substanz in fixer Bauweise in das innere Lumen des lateralen Schenkels des T-Stücks integriert ist,

Fig. 19    einen Querschnitt durch eine mehrlagige Ballonfolie zur Herstellung einer schlauchartigen Struktur und/oder eines Behälters zur Aufnahme von zu- und/oder abzuleitenden Substanzen und/oder einer Ballonkomponente für eine Kopfeinheit einer katheterartigen Vorrichtung,

Fig. 20    eine der Fig. 19 entsprechende Darstellung einer abgewandelten Ausführungsform der Erfindung,

Fig. 21    eine der Fig. 19 entsprechende Darstellung einer andern Ausführungsform der Erfindung, sowie

Fig. 22    eine der Fig. 21 entsprechende Darstellung einer weiter entwickeloten Ausführungsform der Erfindung.

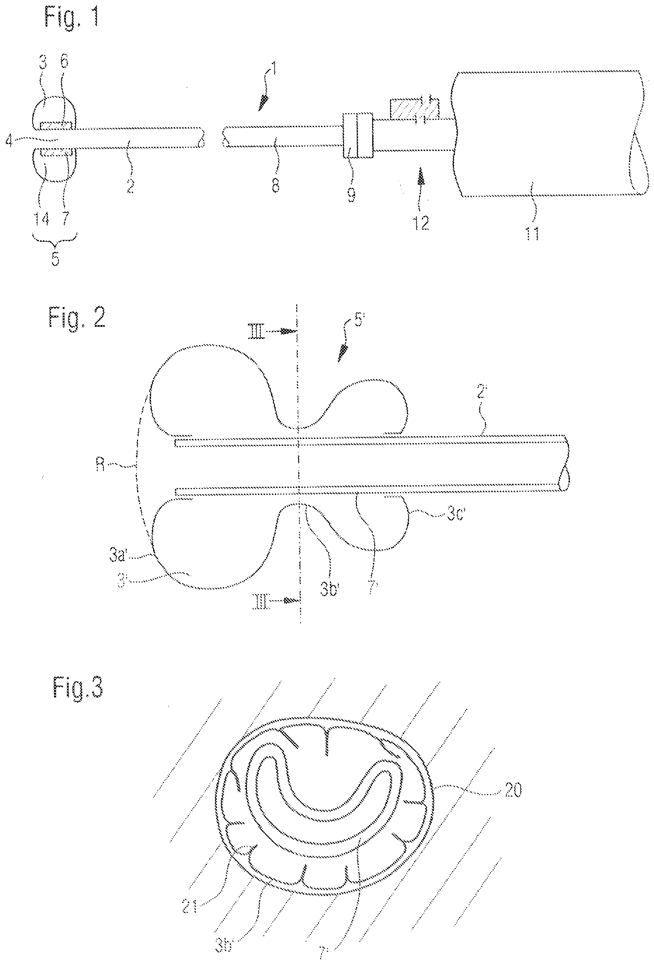

Fig. 1 zeigt den schematischen Aufbau einer medizinischen, katheterartigen Vorrichtung 1 für die zur Umgebung des Patienten hin dekomprimierende, geruchsreduzierende oder geruchsvermeidende Zu- und/oder Ableitung von Substanzen zu/aus dem menschlichen Körper, welche in der Figur am Beispiel einer kontinuierlich im Rektum verweilenden Vorrichtung 1 zur transanalen Ableitung von Stuhl in einen extrakorporal angeordneten Behälter 11 erläutert wird.

Die Kathetervorrichtung 1 umfasst eine schlauchfolienartige Struktur 2, eine am distalen, rektal positionierten Ende der Kathetervorrichtung 1 angeordnete, stuhlaufnehmende Kopfeinheit 5, die einen im Rektum ankernd (retinierend) wirkenden Ballonkörper 3 aufweist.

Besonders einfache Ausführungen der Kopfeinheit 5 verfügen über einen toroidal-ringförmigen Ballonkörper 3, bei dem die zentrale, stuhlaufnehmende Mündung 4 durch ein ring- oder hülsenartiges Element 6 offengehalten wird. Dieses Element 6 gewährleistet, dass es bei der Beaufschlagung des Ballons 3 mit Fülldruck zu keinem radial nach innen gerichteten Kollaps des den Ballon 3 tragenden Abschnitts 7 des Schlauchs 2 bzw. zum Verschluss der zu- und/oder ableitenden Öffnung 4 zum jeweiligen Binnenraum des Körpers kommt. Das mit dem ballontragenden Schlauchabschnitt 7 flächig verbundene, hülsenartige Element 6 begegnet dieser nach innen wirkenden Kraft durch eine elastische, nach außen wirkende Selbstaufrichtung des ring- oder hülsenartigen Elementes 6.

Im Gegensatz zu relativ dickwandigen, elastisch auf ein zu erreichendes Arbeitsmaß aufgedehnten Ballonkomponenten aus Silikon, ermöglichen nicht- oder nur gering volumendehnbare Materialien, wie beispielsweise thermoplastische Polyurethane (TPU) im Härtebereich von Shore 80A bis 95A oder 55D bis 65D zum einen die vollständige, initiale Ausformung des Ballonkorpus auf sein erforderliches Arbeitsmaß bei der Herstellung, zum anderen können sehr niedrige Wandstärken im Bereich weniger Mikrometer, von beispielsweise etwa 10 bis 30 $\mu$m, erreicht werden. Die Verwendung von thermoplastischem Polyurethan (TPU) ermöglicht die Herstellung besonders dünnwandiger, komplex ausgeformter Ballonkomponenten 3, die sich, bedingt durch die niedrige Material-Compliance (Volumen-Dehnbarkeit) der eingesetzten TPU-Typen, trotz niedrigster Wandstärken, auch bei hohen, im Körper auf den Ballon 3 einwirkenden Kräften, verlässlich formstabil verhalten. Da eine elastische. Aufdehnung der Ballonwandung nicht erforderlich ist, können die für die spezifische Funktion im Körper erforderlichen Fülldrucke relativ niedrig sein und sich beispielsweise im Falle der rektalen Stuhlableitung, dem im Rektum herrschenden physiologischen Organdruck nähern. Die Option einer nahezu druckneutralen Befüllung bietet sich insbesondere bei einer inkompletten, schlaff spannungslosen Befüllung des Ballons 3.

Am proximalen Ende 8 des schlauchartigen Segments 2 ist ein Konnektor 9 vorgesehen, woran wahlweise der beutelartige Behälter 11 oder eine Entgasunsg-Einrichtung 12 anschließbar ist.

Fig. 2 zeigt eine Ausführung einer rektal stuhlaufnehmenden und trans-anal durchleitenden Kopfeinheit 5', umfassend eine hantelförmig ausgeformte Ballonkomponente 3', welche einem elastisch verformbaren, sich spontanelastisch in seine Ausgangsform aufrichtenden, zylindrischen oder schlauchförmigen Innenkörper 6' oder dem distalen Schlauchsegment 7' aufsitzt. Die hantelförmige Ballonkomponente 3' umfasst mehrere Segmente 3a' bis 3c', wobei das mittige Segment 3b' gegenüber den beiden endständigen Segmenten 3a', 3c' verjüngt ist. Diese Gestalt der Ballonkomponente 3' wird bereits bei der Herstellung auf das vollständige, für die rektale Retention erforderliche Maß ausgeformt. Die mittige Verjüngung 3b' wird innerhalb des Analkanals platziert, wobei die distal endständige Erweiterung 3a' der Hantel im Rektum und die proximal endständige Erweiterung 3c' unmittelbar prä-anal zu liegen kommt. Die zum Rektum gerichtete Mündung 4' des schlauchförmigen Innenkörpers 6' überragt den distalen Radius R der auf dem Innenkörper 6' montierten Balionkomponente 3' im befüllten Zustand nicht. Die von extrakorporal befüllbare Kopfeinheit 5' geht nach proximal in einen stuhlableitenden Schlauchanteil 2 über, der die Kopfeinheit 5' mit einem extrakorporalen Behälter 11 oder einer zwischengeschalteten Entgasungseinheit 12 verbindet.

Fig. 3 zeigt einen Schnitt durch die Anordnung gemäß Fig. 2 entlang der Linie III - III der in situ liegenden Kopfeinheit 5', derart, dass der taillierte Abschnitt 3b' im Analkanal 20 liegt, Wie man erkennen kann, bilden sich dabei Invaginationen 21 des betreffenden Abschnitts 3b' der Ballonhülle in Form von faltenartigen Strukturen, wie sie sich bei einem residual, d.h. auf ein Übermaß ausgeformten, dichtenden und/oder tamponierenden Ballonkörper 3' bei der Platzierung innerhalb eines in Relation zu dem residual bemessenen Ballon kleineren Lumens 20 durch Einstülpung der überschüssigen Ballonwandung entwickeln.

Die Invaginationen 21 weisen dabei jeweils einen stegartigen, flächig geschlossenen Anteil auf, während sich am blinden, zum Ballonzentrum gerichteten Ende jeder Invagination eine ösenartige Formation ausbildet. Im Bereich der sich formierenden Öse schlägt die Wandung der Ballonhülle um 180 Grad um, wobei durch die elastischen Aufrichtungseigenschaften der in die Wandung integrierten Folienlage eine ausgeprägte, öffnende Wirkung auf die ösenartige Formation generiert wird. Abhängig von der Größe der Querschnittsfläche der jeweiligen ösenartigen Formation, sowie von einer größtmöglichen Vermeidung bzw. Reduktion von zyklischen Kalibersprüngen der Öse, resultiert die jeweilige, zu einem bestimmten Zeitpunkt effektive Dichtungswirkung des Ballons. Kleine Querschnittflächen der Öse wirken sich aufgrund von Kapillareffekten auf Sekrete, die sich innerhalb der Öse befinden, in flusshemmender Weise bis hin zur vollständigen Stase des Sekretes bzw. des Öseninhaltes aus. Der den freien Durchfluss von Sekret hemmende Effekt geht mit zunehmender Weitung bzw. Vergrößerung der Querschnittfläche der Öse verloren.

Die dichtungsrelevante Querschnittsfläche der ösenartigen Formation wird neben der jeweiligen Eigenschaft zur elastischen Aufrichtung der ösenartig umgeschlagenen Ballonwandung durch den jeweils aktuell im Ballon herrschenden Fülldruck bestimmt, der insbesondere den beiden . Wandungslagen des stegartigen Anteils der Invagination. 21 anliegt, und diese flächig, in dicht schließender Weise aneinander presst, wobei im Bereich des Umschlags der beiden Wandungslagen, d.h. an dem blinden Ende der betreffenden Invagination, ein offenes Lumen verbleibt.

Im Analkanal 20 verursacht der normale Tonus des Sphinktermuskels, dass das innere Lumen der beiden in der Ebene III - III konzentrischen Strukturen 7', 3b', nämlich der Schlauchanteil 7', von seiner präformierten kreisrunden Form kollabiert bis zu einem eingefalteten Profil. Dadurch wird eine permanente Öffnung und Dehnung des Sphinktermuskels vermieden. Gleichzeitig bleibt die äußere Struktur, nämlich das transanale Ballonsegment 3b', in dichtendem Kontakt mit der Analschleimhaut, wobei die Kraft, welche die Ballonhülle gegen die innere Oberfläche des Analkanals 20 presst, innerhalb des Rektums aufgenommen wird und dabei den aktuellen Analkräften folgt.

Sobald der Analtonus absinkt und/oder der Analkanal sich öffnet, richtet sich der innere Schaft 7' wieder auf und öffnet das zentrale Lumen. Dabei übertrifft die elastische Selbstaufrichtungskraft die von dem mit Fülldruck beaufschlagten Ballon auf den Schaft ausgeübte Kraft. Das Lumen innerhalb des Schaftes soll bei einem Fülldruck von 25 mbar nicht auf 50% seines maximalen Querschnitts kollabieren.

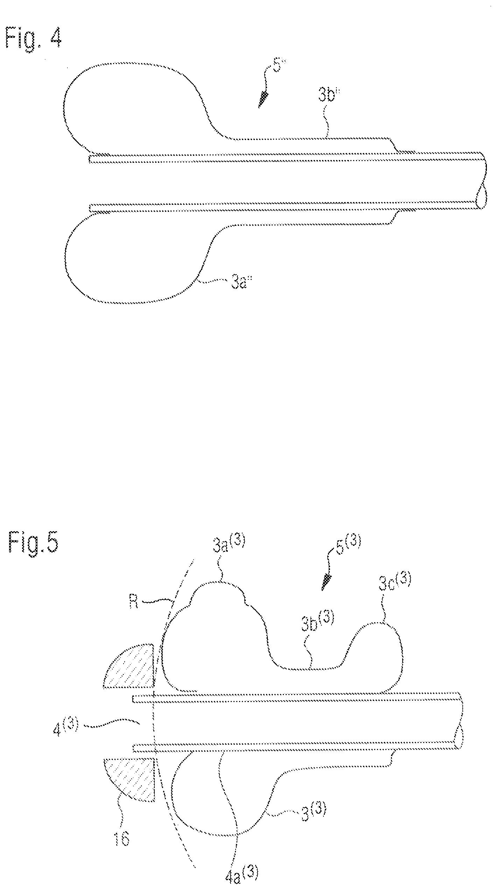

Fig. 4 zeigt eine von Fig. 2 abgeleitete Bauform einer Kopfeinheit 5", wobei die Ballonkomponente 3" eine pilzförmige Gestalt aufweist und auf eine proxinmal endständige, prä-anale Erweiterung verzichtet, sondern nur einen distalen, radial erweiterten Bereich 3a" und einen proximal daran anschließenden, radial verjüngten Bereich 3b" aufweist. Das proximale, trans-anal positionierte Segment 3b" reicht optional in den Analkanal hinein, durch den Analkanal hindurch, oder ragt über die Öffnung des Anus hinaus.

Fig. 5 enthält eine ebenfalls von den Figuren 2 und 4 abgeleitete Bauform einer Kopfeinheit $5^{(3)}$ mit einer Ballonkomponente $3^{(3)}$, bei der die zum Rektum gerichtete Mündung $4^{(3)}$ des Innenkörpers $6^{(3)}$ oder des distalen Schlauchsegments $7^{(3)}$ den distalen Radius R der auf dem Innenkörper $4a^{(3)}$ montierten Ballonkomponente $3^{(3)}$ im befüllten Zustand überragt. Das frei in das Rektum hinein reichende Ende des des Innenkörpers $6^{(3)}$ oder des distalen Schlauchsegments $7^{(3)}$ ist dabei vorzugsweise von einem besonders weich ausgeführten, kappenartigen oder olivenartigen Element 16 in nach lateral und frontal schützender Weise eingefasst.

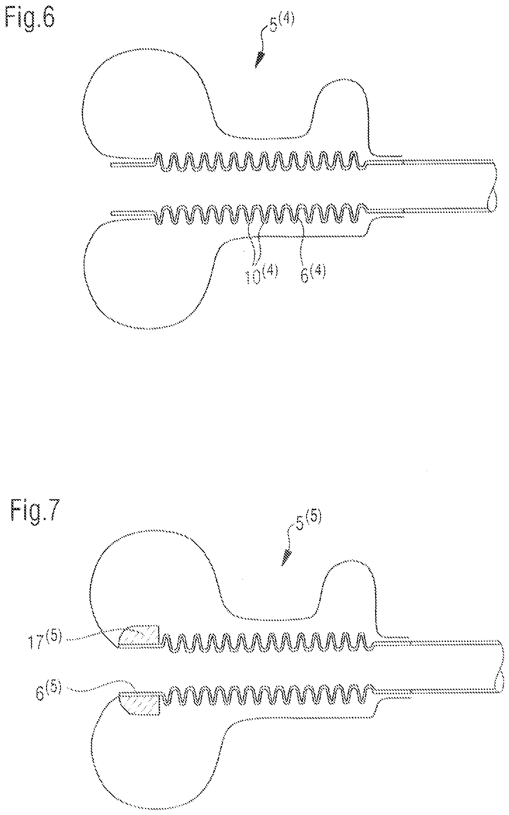

Fig. 6 offenbart eine wiederum abgewandelte Bauform einer erfindungsgemäßen Kopfeinheit $5^{(4)}$, wobei der den hantel- oder pilzförmigen Ballon $3^{(4)}$ tragende Innenkörper $6^{(4)}$ ein welliges, ring- oder wendelartig ausgeformtes Profil $10^{(4)}$ aufweist, welches sowohl im rektalen als auch im trans-analen Segment, als auch in beiden Segmenten der Kopfeinheit $5^{(4)}$ integriert sein kann und so die elastischen Verformungs- und Aufrichtungseigenschaften des ballontragenden Innenkörpers $6^{(4)}$ vorteilhaft modifiziert, indem die radiale Verformung und Aufrichtung in einer beschleunigten, besonders prompten Art und Weise erfolgt. Die baulich geometrische Ausführung welligen Profils $10^{(4)}$ kann dabei segmentspezifisch variiert werden. Beispielsweise kann das Wellungsprofil $10^{(4)}$ im Bereich des rektalen positionierten Segments der Kopfeinheit $5^{(4)}$ derart ausgeformt sein, dass die lumenerhaltende bzw. -aufrichtende Wirkung der Wellung $10^{(4)}$ die entsprechende Wirkung im trans-analen Segment der Kopfeinheit $5^{(4)}$ überwiegt, was im rektalen Segment dazu beiträgt, den Mündungsbereich zum Rektum zu stabilisieren und im trans-analen Segment die Verformungs- und Aufrichtungseigenschaften derart einstellt, dass sich der Schaftinnenkörper $6^{(4)}$ bei normalem Schließmuskeltonus radial einstülpt und sich bei nachlassendem Tonus prompt elastisch in den sich öffnenden Analkanal hinein entwickelt.

Fig. 7 zeigt eine aus Fig. 5 abgeleitete Bauform einer erfindungsgemäßen Kopfeinheit $5^{(5)}$, bei der das rektale Segment des Innenkörpers $6^{(5)}$ durch ein lumenstabilisierendes, ring- oder hülsenartiges Element $17^{(5)}$ erfindungsgemäßen Kopfeinheit $5^{(5)}$ ergänzt wird, welches flächig mit der Innen- oder Außenfläche des rektalen Segmentes des rohrförmigen Innenkörpers $6^{(5)}$ verbunden ist.

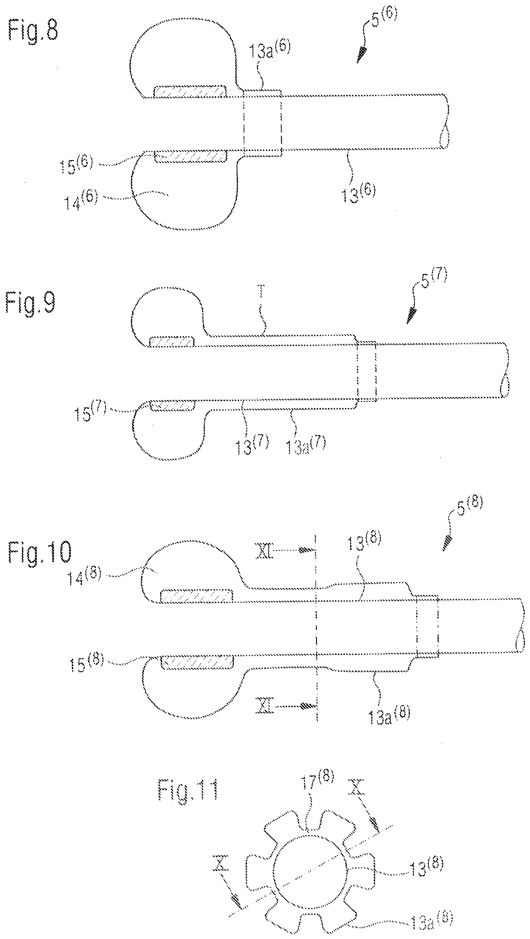

Fig. 8 zeigt eine besonders einfache Bauform einer stuhlableitenden Kopfeinheit $5^{(6)}$, wobei das eine verjüngte Ende $13^{(6)}$ einer sphärisch oder diskoid ausgeformten Ballonschlauchkomponente durch das andere verjüngte Ende $13a^{(6)}$ hindurchgestülpt ist und die beiden Enden $13^{(6)}$, $13a^{(6)}$ miteinender flächig, dicht schließend zu einem befüllbaren

Kompartiment 14$^{(6)}$ verbunden sind, welches somit einen in einem Körperbinnenraum platzierbaren, retinierend wirkenden Ballonanker ausbildet. Zur Stabilisierung der stuhlaufnehmenden Mündung ist das rektale Segment mit einem ring- oder hülsenartigen Element 15$^{(6)}$ ausgestattet.

Fig. 9 gibt eine aus Fig. 8 abgeleitete Bauform einer stuhlableitenden Kopfeinheit 5$^{(7)}$ wieder, bei welcher die beiden verjüngten Ballonenden 13$^{(7)}$ und 13a$^{(7)}$ in den Analkanal hinein, oder auch durch,durch ihn hindurchgeführt sind. Im trans-analen Segment T liegen die beiden Ballonenden 13$^{(7)}$, 13a$^{(7)}$ dann in konzentrischer Anordnung. Im trans-analen Segment T ist kein weiteres, lumenaufrichtendes Element verbaut. Auch hier kann zur Stabilisierung der stuhlaufnehmenden Mündung das rektale Segment mit einem ring- oder hülsenartigen Element 15$^{(7)}$ versehen sein.

Fig. 10 zeigt eine von Fig. 9 abgeleitete Ausführungsform einer stuhlableitenden Kopfeinheit 5$^{(8)}$. Wie man der Schnittdarstellung in Fig. 11 entnehmen kann, sind bei dieser Ausführungsform die beiden konzentrischen, trans-anal positionierten Ballonenden 13$^{(8)}$, 13a$^{(8)}$ durch gleichmäßig über den Umfang des Segmentes verteilte, längsverlaufende, stegartige Schweißungen 17$^{(8)}$ miteinander verbunden. Kommt es zu einer Verschiebung von Volumen aus dem rektalen Segment des befüllbaren Kompartimentes 14$^{(8)}$ in das transanale Segment, richtet sich dieses luftmatratzenartig auf und gibt somit das zu- und oder ableitende Lumen frei. Wiederum kann zur Stabilisierung der stuhlaufnehmenden Mündung das rektale Segment mit einem ring- oder hülsenartigen Element 15$^{(8)}$ versehen sein.

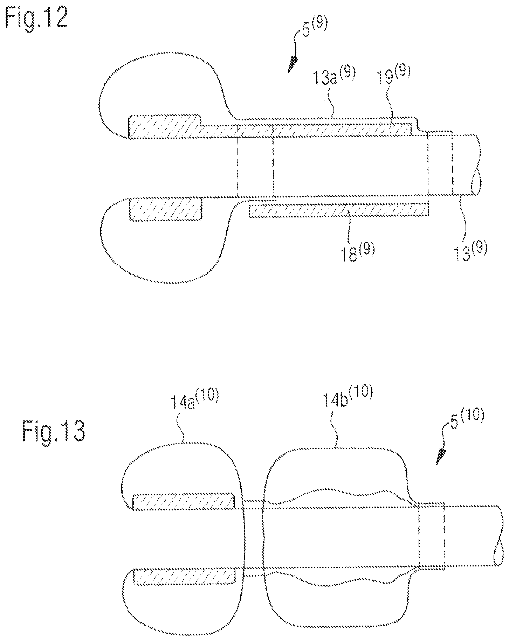

Fig. 12 zeigt eine eine stuhlableitende Kopfeinheit 5$^{(9)}$ als bauliche Ergänzung zu Fig. 10, bei der im trans-analen Segment ein oder mehrere, elastisch verformbare, lumenstabilisierende, hülsen- oder ringartige Komponenten 18$^{(9)}$, 19$^{(9)}$ auf der Außenfläche und/oder Innenfläche der inneren der beiden konzentrischen Lagen 13$^{(9)}$, 13a$^{(9)}$ verbaut sind, die das trans-anale Segment zum einen in den sich Offenen Schließmuskel hinein aufrichten, zum anderen dem im befüllbaren Kompartiment herrschenden Fülldruck wiederstehen und einem nicht erwünschten Kollaps des zu- und/oder ableitenden Lumens vorbeugen.

Fig. 13 stellt eine weitere Bauform einer Kopfeinheit 5$^{(10)}$ dar, bei der das rektale und das trans-anale Segment strukturell separiert sind bzw. sich zwei separat befüllbare Kompartimente 14a$^{(10)}$ und 14b$^{(10)}$ seriell aneinander reihen, wobei das trans-anale Segment bevorzugt aus zwei konzentrischen, endständig zum geschlossenen, befüllbaren Raum verschweißten Schlauchlagen aufgebaut ist, und wobei das rektal platzierte Kompartiment 14a$^{(10)}$ eine die Kopfeinheit 5$^{(10)}$ ankernde, und das trans-anale Segment eine den Analkanal dichtende und gleichzeitg das zu- und/oder ableitende Lumen verschließende Funktion haben. Diese besondere Bauform gestattet bei passagerer Befüllung des trans-analen Kompartiments eine besonders kräftige, effiziente trans-anale Dichtuhgsleistung. Wird das Kompartiment evakuiert liegen die beiden schlauchlagen einander dicht an, und geben das Lumen der Kopfeinheit vollständig frei.

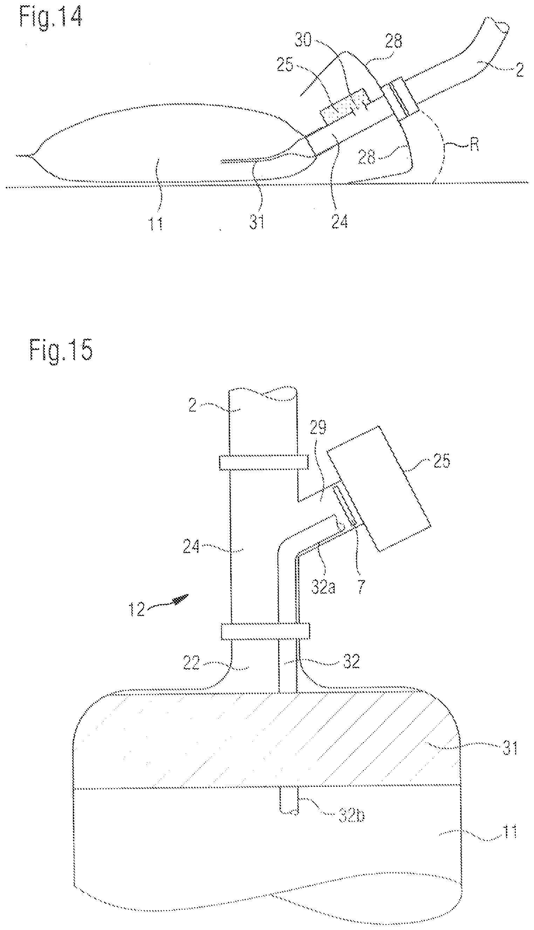

Fig. 14 zeigt eine Ausführungsform eines beutelartigen Behälters 11, wobei in horizontaler Lage des Behälters 11 der Ausstrom von Beutelinhalt in das zuleitende Segment 24 des Behälters 11 - welches vorzugsweise mit der Entgasungs-Einrichtung 12 integriert ist - durch eine rampenartige Steigung R des zuleitenden Segmentes 24 erschwert, reduziert bzw. verhindert wird. Hierzu wird der winkelförmige Haken 28 zur vertikal hängenden Befestigung des Beutels 11 derart ausgeführt, dass er das zuleitende bzw. auch gasablassende Segment 24, relativ zum flach liegenden beutelartigen Behälter 11, in eine schräge, aufsteigend gerichtete Position bringt. Das entstehende Gefälle gewährleistet, dass flüssiger Beutelinhalt, über eine gewisse Phase der Flachlagerung des Beutels 11 hinweg, die gasablassende Öffnung 30 im zuleitenden Segment 24 des Behälters 11 möglichst nicht erreicht. Der winkelförmige Haken 28 ist bevorzugt doppelseitig, zu beiden Flachseiten eines beispielsweise aus zwei Lagen verschweißten, beutelförmigen Behälters 11 hin ausgeführt, so dass sich die erwünschte Schräge in jedem Falle der Flachlagerung des Beutels 11 einstellt. Der Haken 28 bzw. Doppelhaken ist in der bevorzugten Bauweise in einem fixen 90°-Winkel zu den Flachseiten des Beutels ausgerichtet. Die für die Schräge erforderliche 90°-Position kann auch durch einen axial rotierbaren, in eine 90°-Position schwenkbaren und dort einrastenden Haltewinkel erreicht werden.

Wie Fig. 14 weiter zeigt, gibt es bei einer bevorzugten Ausführungsform der Erfindung zwischen dem Behälter 11 und der Zuleitung 24 bzw. der vorgeschalteten Entgasungs-Einrichtung 12 eine ventilartig flussrichtende Funktion, insbesondere in Form einer Doppellage von Folien 31, welche einen Rückstrom von geformten Stuhlanteilen aus dem Behälter 11 in das zum Beutel zuleitende Segement 24 vermeiden soll.

Man erkennt insbesondere zwei einander flächig anliegende Folienlagen 31, die im Inneren des Behälters 11 verbaut sind. Zum Sammelgefäß 11 ablaufendes Material tritt durch die beiden Folienlagen 31 hindurch in den sammelnden Raum des Behälters 11 ein. Füllt sich der Behälter 11 in der vertikal hängenden oder stehenden Normalposition bis auf Höhe der beiden Folienlagen 31 bzw. über diese hinaus, verursacht der flüssige Inhalt einen lippenartigen Schluss der Folienalgen. und der ungewünschte Ausstrom bzw. Rückstrom von Inhalt aus dem Sammelbehälter 11 durch die zuleitende Öffnung wird verhindert. Ein entsprechender Effekt stellt sich ein, wenn gasförmige Substanzen in das Sammelgefäß gelangen. Die Gasfüllung führt ebenfalls zu einem dichten Verschluss der beiden Folienlagen 31. wodurch sich das Gas sukzessive im Behälter 11 staut und der schließlich resultierende Verschlussdruck auf die beiden Folienlagen 31 derart ausgeprägt ist, dass ein weiterer Einstrom von flüssigem Inhalt in das sammelnde Gefäß 11 nicht mehr möglich ist. Ohne entsprechende bauliche Modifikation der Anti-Rückschlag Funktion, muss das Sammelgefäß 11 in diesem Falle gewechselt werden. Um Gas, welches bereits in den Beutel 11 vorgedrungen ist, aus dem Beutel 11 abzuleiten, sieht die Erfindung speziell ausgeführte Folienlagen 31 vor, die zwar flüssigkeitsdicht sind, jedoch Gase frei passieren lassen. Die Folienlagen können flächig, z.B. vollständig aus einem entsprechenden Material bestehen, oder die entsprechenden Eigenschaften auch nur anteilig in bestimmten Segmenten aufweisen. Vorteilhaft wird die gasdurchlässige Funktion oder Einheit dort platziert, wo ein vollständiger Verschluss der beiden Folienlagen 31 auch bei maximaler Füllung des Gefäßes mechanisch bedingt ausgeschlossen ist. Dies ist im Bereich der Anbindung bzw. des Übergangs der Folienlagen zu einem rohr- oder stutzenartigen Einlauf- oder Konnektorsegment 22 gegeben, wo die dichtenden Folienlagen 31 beispielsweise angeschweißt oder angeklebt sind. Bei einem Außendurchmesser des zuleitenden Segments 24 von ca. 15 bis 25 mm stellt sich i.d.R. ein zwickeliörmiger, offener, auch bei maximaler Füllung des Gefäßes nicht schließender Bereich ein, der eine Höhe von mindestens 5 bis 15 mm aufweist, und somit ausreichend Fläche für die beschriebene Funktion bzw. Integration einer "gasdurchlässigen Trennlage" bietet.

Alternativ zu einem solchen gasdurchlässigem Folienmaterial, kann in diesem zwickelförmigen Bereich auch eine kleine, freie Öffnung von beispielsweise 1 bis 2 mm Durchmesser positioniert sein. Im bevorzugten Falle wird jedoch im beschriebenen zwickelförmigen Bereich eine einfache oder kreuzförmige Schlitzung mit einer scharfen Klinge durchgeführt, wobei die Schenkel der Schlitzung jeweils etwa 5 mm Länge aufweisen. Die Schlitzung kann zwar den Rückstrom von Inhalt in das Segment oberhalb des sammelnden Raumes nicht vollständig verhindern, schließt aber einen höhervolumigen Rückstrom von bereits gesammeltem Inhalt aus.

Ferner zeigt die Fig. 15 eine schlauch- oder rohrartige, flexibel oder starr ausgeführte Komponente 32, deren zum Patienten gewandtes Ende 32a fest an der Innenwandung eines zuleitenden Grundelementes 24, bevorzugt im Bereich eines von dem durchleitungslumen 24 seitlich abzweigenden Schenkels 29 angeordnet ist, und deren proximales Ende 32b über das untere Ende des Grundelementes 24 hinaus, in den stuhlsammelnden. Raum des Sammelgefäßes 11 eintaucht. Die Komponente 32 überwindet dabei rückschlaghemmende Komponenten, wie beispielsweise lappenartige, einander dicht schließend anliegende und ventilartig wirkende Folienlagen 31, so dass in den Beutel eingedrungenes Darmgas durch das Lumen der Komponente 32 hindurch, und/oder an der Außenseite des Komponente entlang, vorzugsweise direkt in den gasablassenden Schenkel 29 der Entgasungs-Einrichtung 12 entleert werden kann.

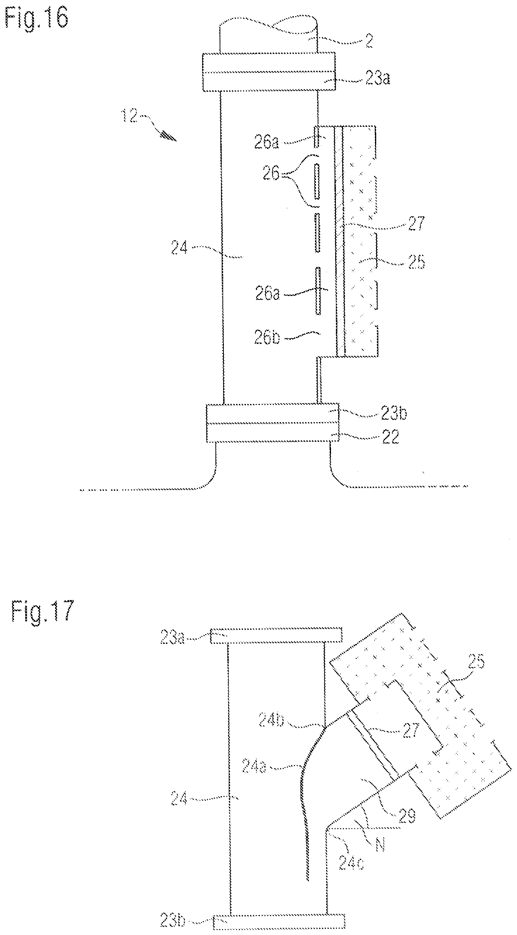

Fig. 16 zeigt eine stuhlableitende, Darmgas zur Umgebung ableitende Einrichtung 12, die als separate, modulare Einheit zwischen dem beutelseitigen Ende 8 der Drainageleitung 2 bzw. einem dort vorgesehenen Konnektor 9 und dem stuhlaufnehmenden Einlass 22 des Sammelbeutels oder Behälters 11 angeordnet ist. Die Entgasungs-Einrichtung 12 verfügt jeweils endständig, zur Drainageleitung 2 und zum Sammelbeutel 11 gerichtet, über konnektierende Komponenten 23a und 23b, mit denen die Entgasungs-Einrichtung 12 in serieller Anordnung, bevorzugt im Bereich des Übergangs von der Drainage zum Sammelbeutel bzw. zum stuhlaufnehmenden Behälter 11, in das stuhldrainierende System eingefügt werden kann. Ein Grundelement 24 verbindet die konnektierenden Komponenten 23a und 23b derart, dass diese in einer miteinander kommunizierenden Verbindung stehen.

An den äußeren Umfang der Wandung des rohrartig zylindrischen Grundelementes 24 der Vorrichtung ist ein entgasendes, adsorbierendes und/oder filterndes Modul 25 in reversibel lösbarer, oder auch in fest mit dem Grundelement 24 verbundener Form angebracht. Das Modul 25 schmiegt sich der Wandung des Grundelementes 24 zirkulär geschlossen als Manschette, oder auch als nicht-geschlossene, C-förmige Schale an. Das Grundelement 24 verfügt im Bereich der Anbindung des Filtermoduls 25 über eine oder mehrere Öffnungen 26, denen sich ein spaltförmiger Zwischenraum 26a anschließt, der zum adsorbierenden bzw. filternden Material weisend, durch eine wasserundurchlässige, aber gasdurchlässige Trennlage 27 begrenzt ist. Die Trennlage 27 kann z.B. aus einem GoreTex-artigen Material bestehen, welches auf der dem Zwischenraum 26a zugewandten Seite durch einen schmutzabweisenden, beispielsweise lotusartigen Effekt ergänzt sein kann. Alternativ kann ein kostengünstiges, HME-Filter-typisches Vliespapier als Trennlage 27 verwendet werden, das ebenfalls wasserabweisend und zugleich luftdurchlässig wirkt. Konzeptionell ist die dem Stuhl zugewandte Seite der Trennlage 27 bevorzugt derart ausgeführt, dass eine Exposition mit Stuhl nicht zum dauerhaften, gasdichten Verschluss der Trennlage 27 führt. Vorzugsweise am unteren, zum Behälter 11 hin gerichteten Ende des Zwischenraumes 26a geht dieser über eine Öffnung 26b in den stuhlführenden bzw. stuhlableitenden Raum des Grundelementes 24 über. Stuhl, der über die Öffnungen 28 in den Zwischenraum 6a gelangt, kann somit über die Öffnung 26b zurück in das Lumen des Grundelementes 24 abfließen. In Abwandlung dieser Bauform kann das stuhlführende Lumen des Grundelementes 24 auch lediglich über die Öffnung 26b mit dem Zwischenraum 26a verbunden sein, also keine Öffnungen 26 aufweisen. Der Zwischenraum 26a erstreckt sich dann von der Öffnung 26b kaminartig nach oben, wodurch die oberhalb der Öffnung 26b liegende Trennlage 27 in senkrechter Normallage der Entgasungs-Einrichtung 12 vor einer direkten Stuhiexposition geschützt ist.

Fig. 17 zeigt eine Ausführungsform mit einem T-Stück-artigen Grundelement 24 mit einem lateralen, sich zur Umgebung hin öffnenden Schenkel 29, an welchem das adsorbierende und/oder filternde Modul 25 aufgebracht ist. Der Schenkel 29 ist in der bevorzugten Ausführung dieser Bauform in einem Neigungswinkel N zur Senkrechten bzw. zur Längsachse des Grundelementes 24 nach oben bzw. in Drainagerichtung nach stromaufwärts gerichtet, von ca. 15 bis 60 Grad, bevorzugt 30 bis 45 Grad. Das Modul 25 ist somit bei einer hängenden, annähernd senkrechten Normallage der Entgasungs-Einrichtung 12 bzw. der Einheit von stuhl-ableitendem Drainageschlauch 2, entgasender Vorrichtung 12 und sammelndem Beutel 11, vor dem direkten Einstrom von Stuhl geschützt. Stuhl, der bereits in den Schenkel 29 eingedrungen ist, kann von dort, der Neigung des Schenkels 29 folgend, in das Lumen des Grundelementes 24 ablaufen.

Ergänzend zur beschriebenen Neigung des entgasenden Schenkels, kann der Übergang zum stuhlführenden Lumen des Grundelementes 24 durch eine Zunge 24a in Form einer vorhang- oder baldachinartigen Struktur geschützt sein, um den Eintritt von Stuhl in den entgasenden Schenkel 29 noch effizienter zu vermeiden. Die Zunge 24a als abschirmende Struktur geht beispielsweise in dem Übergangsbereich 24b zwischen dem nach lateral weisenden Schenkel 29 und dem senkrecht nach oben bzw. nach stromaufwärts verlaufenden Schenkel des . T-förmigen Grundelements 24 aus der Innenwandung des Grundelementes 24 hervor, und reicht über den unteren Übergang 24c des nach lateral weisenden Schenkels 29 herab, bis in den senkrecht nach unten bzw. in Drainagerichtung nach stromabwärts verlaufenden Schenkel des T-förmigen Grundelements 4 hinein.

Alternativ zur Positionierung einer modularen Vorrichtung 25 zwischen Drainageleitung 2 und Sammelbeutel 11 als eine im Bedarfsfall seriell zuschaltbare Funktionseinheit, kann das Grundelement 24 auch direkt in den oberen Einlassbereich 22 des Sammelgefäßes 11 in fixer Bauart integriert sein. Im Falle eines beutelartigen Containers 11 wird dessen Folienkörper durch eine direkte Verschweißung oder Klebung mit dem nach unten gerichteten, zum Beutel 11 weisenden Schenkel des T-Stücks dicht schließend verbunden.

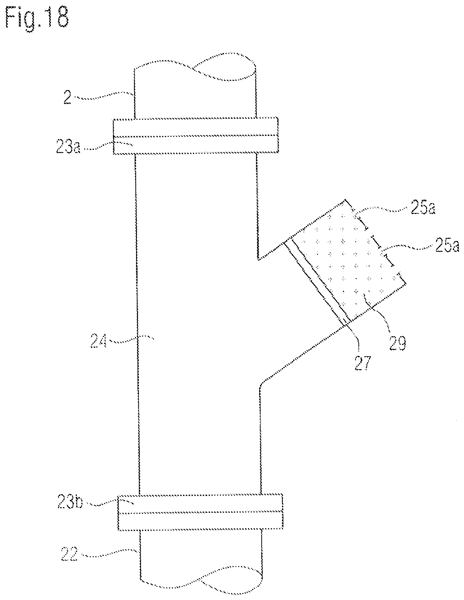

Fig. 18 zeigt eine an Fig.17 angelehnte Ausführungsform der Entgasungs-Einrichtung 12, bei der das adsorbierende und/oder filternde Material in fixer Bauweise bereits in den lateralen, das Gesamtsystem von Drainageleitung 2 und Beutel 11 zur Umgebung hin öffnenden, gasableitenden Schenkel 29 des T-Stück-förmigen Grundelements 24 integriert ist. Der Schenkel 29 ist dabei derart bemessen, dass er z.B. ca. 5 bis 10 ml Aktivkohle-Granulat aufnimmt. Das Kohlegranulat wird zum stuhlführenden, oberen und unteren Schenkel des T-Stück-förmigen Grundelements 24 durch eine gasdurchlässige, wasserabweisende Trennlage 27 abgegrenzt. Die stirnseitige Öffnung des Schenkels 29 ist mit einem kappenartigeri Verschluss versehen, der über Öffnungen 25a für den Gasauslass zur Umgebung verfügt.

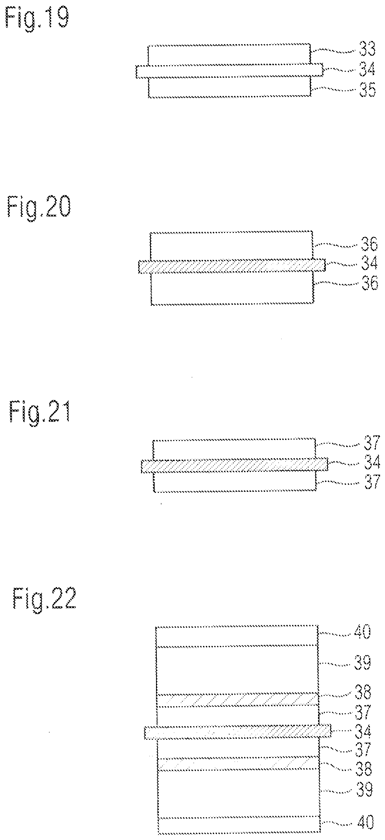

Fig. 19 zeigt einen beispielhaften Wandungsaufbau der katheterartigen Vorrichtung 1, die eingesetzten Materialtypen und deren spezifische, physikalisch-chemische Eigenschaften zum einen im Sinne einer effizienten und körperverträglichen Funktion des Katheters, und zum anderen im Sinne einer effizienten Geruchsdichtung kombiniert. Die Gesamtwandungsstärke der für die Herstellung der Vorrichtung eingesetzten Folie, sowie die Materialhärte und die Stärke der Einzellagen der Folie sind hierbei für die Anforderungen eines stuhlableitenden, dauerhaft im Rektum des Patienten verbleibenden Kathetersystems ausgelegt.

Im Bereich des intra- und extrakorporalen, stuhlableitenden Schlauches, optional auch im Bereich des Ballonkörpers, besteht das eingesetzte Folienmaterial beispielsweise aus einer ersten Trägerlage 33 aus Polyurethan (PUR), gefolgt von einer mittig angeordneten Barrierelage 34 aus Ethylen-Vinylalkohol-Copolymer (EVOH) oder, weniger bevorzugt, auch aus Polyvinylidenchlorid (PVDC). Jenseits dieser Barrierelage 34 schließt sich sodann eine zweite Trägerlage 35, bspw. aus Poyvinylchlorid (PVC) an.

Die Gesamtstärke der Schlauchwandung liegt bei 200 bis 400. $\mu$m, bevorzugt bei 250 bis 350 $\mu$m. Der PUR-Anteil 33 wird bevorzugt nach außen hin angeordnet und weist beispielsweise eine Lagenstärke von 200 $\mu$m auf, seine Materialhärte liegt im Bereich von Shore 80A bis 90A. Das PUR verleiht dem Schlauchkörper 2, aber auch den anderen damit ausgeführten Komponenten, zum einen Zug- und Reißfestigkeit. Zum anderen stattet der PUR-Anteil 33 die Schlauchfolie mit der Fähigkeit zur elastischen Verformung und Selbstaufrichtung aus. Die nachfolgende, im Lagenverbund mittig angeordnete Barrierelage 34 weist eine Wandungsstärke von ca. 15$\mu$m auf, sie besteht bevorzugt aus EVOH. Die Barriere-Schicht 34 minimiert den Durchtritt von Wassermolekülen, Luftbestandteilen und geruchsintensiven Substanzen durch die Folienwandung. Die bevorzugt nach innen, zum Schlauchlumen hin gerichtete zweite Trägerlage 35 besteht aus PVC der Shore Härte 60A bis 80A, und weist eine Lagenstärke von ca. 100pm auf. Sie gewährleistet eine gewisse Barriere gegen Wassermoleküle, die die mittig angeordnete EVOH-Lage bis zu einem gewissen Grad von Wasser abgeschirmt, wodurch der Barriere-Effekt des EVOH nicht durch die Interaktion mit Wassermolekülen beeinträchtigt bzw. reduziert wird.

Um die in den vorausgehenden Figuren beschriebenen Bauformen der Vorrichtung herzustellen, bei denen sowohl der im Rektum retinierende Ballonanteil als auch das trans-anale und/oder das extra-korporale, stuhlableitende Schlauchsegment aus einem einzigen Schlauchrohling ausgeformt werden, schlägt die Erfindung eine angepasste Verteilung der oben genannten, anteiligen Materialstärken vor. Die PUR-Lage 33 wird auf 280 $\mu$m, die EVOH oder PVDC-Lage 34 wird auf 40 $\mu$m verstärkt. Zur Abschirmung des EVOH nach innen, wird eine PVC-Lage 35 in reduzierter Stärke von ca. 80 $\mu$m verwendet. Die solchermaßen dimensionierte Anpassung der PUR-Lagenstärke 33 ermöglicht die geometrisch stabile, symmetrische Blasformung der rektalen, ballonartigen Erweiterung aus dem in den Umformungsprozess eingesetzten, relativ zum Ballondurchmesser kleineren Grundschlauch. Bei einem angenommenen Kalibersprung des Schlauchdurchmessers von 20-30 mm auf einen Ballondurchmesser von etwa 60-70 mm, verdünnt sich die EVOH-Lage 35 im Verlauf einer Blasformung von 40$\mu$m auf etwa 10 bis 15 $\mu$m, was den Erhalt der Barrierefunktion im Ballon sicherstellt bzw. eine kritische Ausdünnung der Barrierelage ausschließt. In entsprechender Weise stellt der höhere PUR-Anteil an der Gesamtwandungsstärke des umzuformenden Rohschlauches sicher, dass der erweitert ausgeformte Ballonanteil eine ausreichende mechanische Belastbarkeit, insbesondere Formstabilität, Reißfestigkeit und Punktionsbeständigkeit aufweist.

Die beschriebene, höhere Wandungsstärke der äußeren PUR-Lage 33 ist insbesondere für die simultane Blasformung des Ballonanteils 3 und eines wellig korrugierten, das Drainagelumen im ballontragenden Bereich 7 stabilisierenden Schaftschlauches 2 aus einem einzigen, durchgängigen . Schlauchrohling vorteilhaft. Die Kombination der zuvor beschriebenen PUR-Typen und deren anteilige Lagenstärke, mit einem spezifisch gewellten, ringartig oder spiralig korrugierten Profil im ballontragenden bzw. vom Ballon 3 eingeschlossenen Abschnitt 7 des Schaftschlauches 2, unterstützt dessen Fähigkeit zur spontanen Aufrichtung in die bei der Herstellung eingestellte Ausgangsform und trägt dazu bei, axial gerichtete Torsionen des Schlauches 2 zu vermeiden.

Fig. 20 offenbart einen Wandungsaufbau eines geruchsdichten, folienartigen Schlauchmaterials, wobei die jeweilige, aus EVOH oder optional auch aus PVDC bestehende Barrierelage 34 beidseitig mit je einer mechanisch stabilen Trägerlage 36 aus Ethylen-Vinylacetat-Copolymer (EVA) kombiniert sein kann.

Fig. 21 beschreibt einen weiteren beispielhaften Wandungsaufbau eines geruchsdichten bzw. die Freisetzung von Geruch hemmenden, folienartigen Schlauchmaterials, wobei die jeweilige, aus EVOH oder optional auch aus PVDC bestehende Barrierelage 34 beidseitig mit je einer mechanisch stabilen . Trägerlage 37 aus Polyamid (PA) oder aus einem thermoplastischen Polyamidelastomer (TPE-A), kombiniert sein kann. Andererseits kann auch eine derartige Trägerlage 37 aus PA oder TPE-A außen mit einer mittigen Barrierelage 34 aus EVOH und einer inneren Lage PVC oder PUR kombiniert werden. Bei einer Gesamtstärke, des in die thermische Umformung zum stuhlableitenden Segment eingesetzten Schlauchfolienrohlings von beispielsweise 300 μm, weist das PA oder TPE-A eine Härte von Shore 35D bis 40D auf und hat eine Lagenstärke von 80 μm. Die mittige EVOH-Lage 34 ist 10 bis 20 μm dick. Der innen liegende PVC- oder PUR-Anteil der Folie hat eine Stärke von 200μm.

Fig. 22 beschreibt einen weiteren mehrlagigen Folienaufbau, wobei eine zentral angeordnete Barrierelage 34 aus EVOH beidseits flankierend durch je eine PA-Lage 37 ergänzt ist. Die beiden Materialien lassen sich in der Regel vorteilhaft einfach im Koextrusionsprozess kombinieren. Über eine KleberLage (Tie-Layer) 38 wird dann eine oder mehrere Lagen eines LDPE-Materials 39, 40 angebunden, beispielsweise zunächst je eine Lage 39 aus einem orientierten Polyethylen niedriger Dichte, und anschließend je einer Lage 40 aus einem normalen Polyethylen niedriger Dichte.

Daneben gibt es auch eine zweilagige Folienkombination, die eine EVOH- oder PVDC-basierte Barriere-Lage lediglich mit einer einzigen Trägerlage kombiniert, also auf eine sandwichartige Lagenanordnung, wie in den vorausgehenden Figuren beschrieben, verzichtet. Die Barriere-Lage weist eine Wandungsstärke von 20 μm auf und wird bevorzugt als Außenfläche angeordnet. Die nach innen gerichtete Trägerlage besteht bevorzugt aus PUR oder PVC und ist beispielhaft 200 bis 280 μm dick. Im Falle einer gleichzeitigen Ausformung sämtlicher Komponenten der Ballon- und Schaftschlauchkomponente der Kopfeinheit 5 sowie des extra-korporalen, stuhlableitenden Schlauches 2 aus einem einzigen eingesetzten Rohling, wird die Stärke der Barriere-Lage auf etwa 50μm, und die anteilige Lagenstärke des Trägers 18 oder 20 auf eine Stärke von 250 bis 300μm angehoben.

Bezugszeichenliste

| | | | |
|---|---|---|---|
| 1 | katheterartige Vorrichtung | 23a | Konnektor |
| 2 | schlauchartige Struktur | 23b | Konnektor |
| 3 | Ballonkörper -segment | 24 | zuleitendes Segment |
| 3a | Ballonsegment | 24a | Zunge |
| 3b | Ballonsegment | 24b | Übergangsbereich |
| 3c | Ballonsegment | 24c | Übergang |
| 4 | Mündung | 25 | Modul |
| 5 | Kopfeinheit . | 25a | Öffnung |
| 6 | Innenkörper | 26 | Öffnung |
| 7 | Schlauchsegment | 26a | Zwischenraum |
| 8 | proximales Schlauchende | 26b | Öffnung |
| 9 | Konnektor | 27 | Trennlage |
| 10 | welliges Profil | 28 | Haken |
| 11 | Behälter | 29 | Schenkel |
| 12 | Entgasungs-Einrichtung | 30 | gasablassende Öffnung |
| 13 | Proximales Schlauchsegment | 31 | Folienlagen |
| 13a | distales Schlauchsegment | 32 | rohrartige Komponente |
| 14 | Erweiterung, Kompartiment | 33 | erste Trägerlage |
| 15 | Hülsenelement | 34 | Barrierelage |
| 16 | olivenartiges Element | 35 | zweite Trägerlage |

(fortgesetzt)

| 17 | Verschweißung | 36 | Trägerlage aus EVA |
|----|---------------|----|---------------------|
| 18 | hülsenartiges Element | 37 | Trägerlage aus PA |
| 19 | hülsenartiges Element | 38 | Kleberlage |
| 20 | Analkanal | 39 | LDPE (orientiert) |
| 21 | Invagination | 40 | LDPE |
| 22 | Konnektor | | |

## Patentansprüche

1. Geschlossenes System für die möglichst geruchsneutrale Zu- und/oder Ableitung von geruchsintensiven Substanzen zu/von dem Körper eines Patienten, umfassend

   - eine in einem Binnenraum des Körpers des Patienten positionierbare, ein ballonartiges Element (3) aufweisende, das System im Körper des Patienten retinierende und/oder dichtende Kopfeinheit (5),
   - einen extrakorporal angeordneten Behälter (11) mit einem bevorzugt folienbasierten, beutelartigen Kompartiment für die Aufnahme der zu- und/oder abzuleitenden Substanzen, sowie
   - eine schlauchfolienartige Struktur (2), die den zu erreichenden Körperbinnenraum mit dem extrakorporal angeordneten Behälter verbindet,

   **dadurch gekennzeichnet, dass** die zu- und/oder ableitende, schlauchfolienartige Struktur (2) des Systems sowie die folienbasierten Bestandteile des Behälters (11) aus mehrlagigem Folienmaterial bestehen, das jeweils wenigstens eine als Geruchsbarriere wirkende Barrierelage (34) aus Ethylen-Vinylalkohol-Copolymer (EVOH) und/oder aus Polyvinylidenchlorid (PVDC) aufweist, sowie jeweils wenigstens eine Trägerlage (35,37,39,49) aus einem robusten, mechanisch belastbaren Material, wobei das Material einer Trägerlage ausgewählt ist aus der Gruppe umfassend Polyurethan (PUR), bevorzugt thermoplastisches Polyurethan (TPU), Polyvinylchlorid (PVC), Polyamid (PA), thermoplastisches Polyamidelastomer (TPE-A) und polyolefin-basierte Polymere, sowie Kombinationen aus diesen Materialien wie bspw. eine Kombination aus einer Trägerlage aus PVC und wenigstens einer Trägerlage aus PUR.

2. System nach Anspruch 1, **dadurch gekennzeichnet, dass** auch die folienbasierten Schlauch- und/oder Ballonbestandteile der im Binnenraum des Körpers positionierten Kopfeinheit (5) aus mehrlagigem Folienmaterial bestehen, das jeweils wenigstens eine als Geruchsbarriere wirkende Barrierelage (34) aus Ethylen-Vinylalkohol-Copolymer (EVOH) und/oder aus Polyvinylidenchlorid (PVDC) aufweist, sowie jeweils wenigstens eine Trägerlage (35,37,39,40) aus einem robusten, mechanisch belastbaren Material.

3. System nach Anspruch 1 oder 2, **dadurch gekennzeichnet, dass** die (Gesamt-) Dicke $d_T$ der Trägerlage oder aller Trägerlagen größer ist als die (Gesamt-) Dicke $d_B$ der Barrierelage oder aller Barrierelagen:

$$d_T > d_B,$$

oder dass die (Gesamt-) Dicke $d_T$ der Trägerlage oder aller Trägerlagen gleich oder größer ist als das Fünffache der (Gesamt-) Dicke $d_B$ der Barrierelage oder aller Barrierelagen:

$$d_T \geq 5 * d_B,$$

oder dass die (Gesamt-) Dicke $d_T$ der Trägerlage oder aller Trägerlagen gleich oder größer ist als das Zehnfache der (Gesamt-) Dicke $d_B$ der Barrierelage oder aller Barrierelagen:

$$d_T \geq 10 * d_B,$$

oder dass die (Gesamt-) Dicke $d_T$ der Trägerlage oder aller Trägerlagen gleich oder größer ist als das Zwanzigfache der (Gesamt-) Dicke $d_B$ der Barrierelage aus EVOH oder PVDC oder aller Barrierelagen aus EVOH und/oder PVDC:

$$d_T \geq 20 * d_B,$$

oder dass die (Gesamt-) Dicke $d_T$ der Trägerlage oder aller Trägerlagen gleich oder größer ist als das Vierzigfache der (Gesamt-) Dicke $d_B$ der Barrierelage aus EVOH oder PVDC oder aller Barrierelagen aus EVOH und/oder PVDC:

$$d_T \geq 40 * d_B.$$

4. System nach einem der vorhergehenden Ansprüche, **dadurch gekennzeichnet, dass** wenigstens eine Trägerlage aus einem mechanisch belastbaren, bevorzugt elastisch verformbaren und sich elastisch aufrichtenden Trägermaterial besteht.

5. System nach einem der vorhergehenden Ansprüche, **dadurch gekennzeichnet, dass** das Material wenigstens einer Trägerlage aus PUR mit einer Shore-Härte von 80A bis 95A oder von 55D bis 65D besteht, und/oder aus einem thermoplastischen PUR von einem Typ mit einer Wasseraufnahme nach DIN ISO 62 von 5% oder weniger besteht, bevorzugt mit einer Wasseraufnahme nach DIN ISO 62 von 2% oder weniger.

6. System nach einem der vorhergehenden Ansprüche, **dadurch gekennzeichnet, dass** wenigstens eine Barrierelage des zwei- oder mehrlagigen Folienmaterials nur an einer Seite mit einer Trägerlage verbunden ist, vorzugsweise wobei die Barrierelage aus PVDC besteht, insbesondere wobei wenigstens eine Trägerlage aus PVC oder PUR besteht.

7. System nach einem der Ansprüche 1 bis 6, **dadurch gekennzeichnet, dass** das mehrlagige Folienmaterial aus einer sandwichartigen Kombination besteht, wobei wenigstens eine mittige Barrierelage an beiden Seiten von wenigstens je einer Trägerlage umgeben ist, vorzugsweise wobei an einer oder beiden Seiten der mittigen Barrierelage eine Trägerlage aus PVC vorgesehen ist, und/oder wobei vorzugsweise zu einer oder beiden Seite der mittigen Barrierelage eine Trägerlage aus PUR vorgesehen ist, und/oder wobei insbesondere an jeder Seite einer mittigen Barrierelage wenigstens eine Trägerlage aus PVC mit wenigstens einer Trägerlage aus PUR kombiniert ist.

8. System nach einem der vorhergehenden Ansprüche, **dadurch gekennzeichnet, dass** die Wandungsstärke des mehrlagigen Folienmaterials in einem Bereich von 10 $\mu$m bis 5 mm liegt, und/oder in einem Bereich von 20 $\mu$m bis 2 mm, beispielsweise in einem Bereich von 50 $\mu$m bis 1 mm, vorzugsweise in einem Bereich von 50 $\mu$m bis 1 mm, insbesondere in einem Bereich von 100 $\mu$m bis 500 $\mu$m.

9. System nach einem der vorhergehenden Ansprüche, **dadurch gekennzeichnet, dass** die Dicke einer Barrierelage in einem Bereich von 5 bis 25 $\mu$m liegt, bevorzugt in einem Bereich von 10 bis 15 $\mu$m.

10. System nach einem der vorhergehenden Ansprüche, **gekennzeichnet durch** folgenden Aufbau des mehrlagigen Folienmaterials:

   - PVC, bspw. mit einer Shore-Härte von 60A bis 80A und einer Dicke von 140 bis 280 $\mu$m an der einem zentralen Lumen zugewandten Innenseite als innere Trägerlage
   - PUR, bspw. mit einer Shore-Härte von 55D und einer Dicke von 50 bis 100$\mu$m an der dem zentralen Lumen abgewandten Außenseite als äußere Trägerlage, sowie

- EVOH oder PVDC mit einer Dicke von 10 bis 20 $\mu$m zwischen der inneren Trägerlage und der äußeren Trägerlage als mittige Barrierelage.

11. System nach einem der vorhergehenden Ansprüche, **dadurch gekennzeichnet, dass** eine oder mehrere Lagen des mehrlagigen Folienmaterials miteinander koextrudiert sind.

12. System nach einem der vorhergehenden Ansprüche, **dadurch gekennzeichnet, dass** wenigstens eine Barrierelage, insbesondere eine Barrierelage aus EVOH, mit einer benachbarten Lage aus PA verbunden ist, insbesondere ohne eine verbindende, adhäsionsunterstützende Tie-Layer-Lage, und/oder dass wenigstens eine Trägerlage, insbesondere eine Trägerlage aus PUR, mit einer benachbarten Lage aus PA verbunden ist, insbesondere ohne eine verbindende, adhäsionsunterstützende Tie-Layer-Lage, und/oder dass eine Lage aus PA durch Einsatz eines Hafvermittlers als Zwischenlage mit einer PEbasierten Lage verbunden ist.

13. System nach einem der vorhergehenden Ansprüche, **dadurch gekennzeichnet, dass** unterschiedliche, separat hergestellte, im System aneinandergrenzende Komponenten, wie Kopfeinheit und schlauchfolienartige Struktur einerseits oder Behälter und schlauchfolienartige Struktur andererseits, miteinander verklebt sind, bspw. indem aneinander grenzende Oberflächen zweier separat hergestellter Komponenten mit einem Lösungsmittel verklebt sind, insbesondere mit einem Lösungsmittel wie Cyclohexanon oder Tetrahydrofuran.

14. System nach einem der vorhergehenden Ansprüche, **dadurch gekennzeichnet, dass** der Ballon und die Leitung aus dem selben mehrlagigen Folienmaterial besteht, und/oder dass der Behälter und die Leitung aus dem selben mehrlagigen Folienmaterial besteht, und/oder dass der Ballon, der Behälter und die Leitung aus dem selben mehrlagigen Folienmaterial besteht, vorzugsweise wobei Komponenten, welche aus dem selben mehrlagigen Folienmaterial bestehen, miteinander integriert und/oder als eine Einheit hergestellt sind.

15. System nach einem der vorhergehenden Ansprüche, **dadurch gekennzeichnet, dass** das mehrlagige Folienmaterial zumindest bereichsweise mit einer spiralförmigen oder mit mehreren, seriell aufeinander folgenden, ringförmigen, nach innen oder außen gerichteten Ein- und/oder Ausstülpungen versehen ist, um die spontane Aufrichtung des betreffenden Folienmaterials nach einer passageren Verformung zu unterstützen, vorzugsweise wobei zirkuläre Erweiterungen oder Reduktionen des mehrlagigen Folienmaterials

a) 3 bis 6 mm breit sind, und/oder
b) im Scheitel 1,0 bis 3 mm, bevorzugt 1,5 bis 2,5 mm tief sind.

16. System nach einem der vorhergehenden Ansprüche, **dadurch gekennzeichnet, dass**

a) der Katheterballon ein radial erweitertes, intrakorporales Ballonsegment aufweist sowie ein demgegenüber radial verjüngtes transostiales Ballonsegment, und/oder
b) dass sowohl der gesamte intra- als auch der gesamte extrakorporale Anteil des Katheterballons aus einem einzigen, strukturell durchgängigen Folienschlauchrohling ausgeformt ist.

17. System nach einem der vorhergehenden Ansprüche, **dadurch gekennzeichnet, dass** mehrere Lagen des mehrlagigen Folienmaterials gemeinsam miteinander in einem nachfolgenden Herstellungsschritt, beispielsweise durch ein hot-molding Verfahren, thermisch umgeformt sind, wobei ein zunächst unbearbeiteter Schlauchrohling durch Beaufschlagung mit Druckluft in eine erhitze Formkavität expandiert, und dort durch anschließende Kühlung des Formwerkzeuges in seiner Form fixiert wird.

18. System nach einem der vorhergehenden Ansprüche, **dadurch gekennzeichnet, dass** gleichzeitig zu der Umformung eines extrakorporalen, stuhlableitenden Schlauchsegmentes zu einem beispielsweise mit lumen-stabilisierenden Ein- und/oder Ausstülpungen versehenen Schlauchmantel auch die Ausformung einer intrakorporalen Ballonkomponente des Katheterballons und/oder einer transostialen Ballonkomponente des Katheterballons in einem einzigen Arbeitsgang erfolgt ist.

19. System nach einem der vorhergehenden Ansprüche, **dadurch gekennzeichnet, dass** sich bei der in situ-Platzierung eines residual, d.h. mit einem entlang des Ballonumfangs überschüssigen Ballonmaterial ausgeformten Ballonkorpus typische, in des Balloninnere invaginierte Einstülpungen (21) der überschüssigen, residualen Ballonhülle formieren, vorzugsweise wobei die in das Balloninnere invaginierten Einstülpungen (21) querschnittlich ösenartigen Umschlagformationen aufweisen, die sich vorzugsweise in Längsrichtung des Ballons (3), also zwischen dessen distaler und proximaler Stirnseite, als kanalartige Formationen erstrecken bzw. fortsetzen, insbesondere wobei die querschnittlich ösenartigen Umschlagformationen, die sich vorzugsweise in Längsrichtung des Ballons (3) als kanalartige Formationen erstrecken bzw. fortsetzen, bei einem Fülldruck des Ballons (3) von 30 mbar einen Öffnungsdurchmesser zwischen 30 μm und 120 μm aufweisen, vorzugsweise einen Ösendurchmesser zwischen 40 μm und 80 μm.

20. System nach einem der vorhergehenden Ansprüche, **dadurch gekennzeichnet, dass** zwischen einer elastisch verformbaren Trägerlage aus PUR und einer nicht elastisch verformbaren Trägerlage, beispielsweise aus PVC, eine gas- und/oder wasserdampfdichte Barrierelage (34) angeordnet ist, vorzugsweise wobei die anteilige Wandstärke der nicht elastisch verformbaren Trägerlage (33,35,36,39,40), beispielsweise aus PVC, größer ist als die anteilige(n) Wandstärke(n) der elastisch verformbaren Trägerlage(n) aus PUR.

21. System nach einem der vorhergehenden Ansprüche, **gekennzeichnet durch** eine Entgasungseinrichtung mit einem Auslass, an welchem ein innerer Überdruck innerhalb des Systems **durch** den Austritt von Gas abgebaut werden kann, und mit einer Adsorptions- und/oder Absorptions- und/oder Filtereinrichtung, welche unerwünschte Stoffe aus dem austretenden Gas filtert, insbesondere Geruchsstoffe, vorzugsweise wobei die Entgasungseinrichtung an einem, die jeweilige zu- und oder ableitende Substanz aufnehmenden Kompartiment des Behälters angeschlossen ist, und/oder wobei vorzugsweise in einem Durchleitungsbereich für die Durchleitung der zu- und/oder abzuleitenden Substanzen eine Abzweigung vorgesehen ist, die als Auslass dient, insbesondere wobei die Adsorptions- und/oder Absorptions- und/oder Filtereinrichtung mit der Abzweigung integriert oder an jener angeschlossen ist, wobei insbesondere die Adsorptions- und/oder Absorptions- und/oder Filtereinrichtung über einen geschützten Zugangsweg in der Abzweigung von dem zu- und/oder ableitenden Lumen des Systems räumlich entfernt angeordnet ist, so dass die adsorbierend bzw. filternd wirkende, entgasende Einheit vor direkter Exposition mit den zu- und/oder abzuleitenden Substanzen geschützt ist, und/oder wobei vorzugsweise die Abzweigung distal zu einem rückschlaghemmenden Element am Einlass des Kompartiments in dem Behälter angeordnet ist, insbesondere wobei das rückschlaghemmende Element folienbasierte, lippen- oder segelartige Komponenten (31) aufweist, vorzugsweise wobei sich der Abstand zwischen den folienbasierten, lippen- oder segelartigen Komponenten (31) in der Hauptströmungsrichtung, also in Richtung von der Drainageleitung (2) zu dem Sammelkompartiment des Sammelbehälters (11), verjüngt.

22. System nach Anspruch 21, **dadurch gekennzeichnet, dass** die Adsorptions- und/oder Absorptions- und/oder Filtereinrichtung (25) zumindest bereichsweise von dem Durchleitungsbereich beabstandet ist, um einen intensiven Kontakt mit den Durchleitungsbereich durchströmendem Substanzen so weit als möglich zu vermeiden, und/oder dass die Längsrichtung des Strömungskanals zwischen der Abzweigung (29) und der Adsorptions- und/oder Absorptions- und/oder Filtereinrichtung mit der Längsrichtung des Durchleitungsbereichs zwischen der Abzweigung (29) und dem Ablaufanschluss einen Winkel von 90° oder weniger als 90° einschließt, beispielsweise einen Winkel von 75° oder weniger, vorzugsweise einen Winkel von 60° oder weniger, insbesondere einen Winkel von 45° oder weniger, oder sogar einen Winkel von 30° oder weniger, und/oder dass der maximale Strömungsquerschnitt in dem Bereich zwischen der Abzweigung (29) und der Adsorptions- und/oder Absorptions- und/oder Filtereinrichtung (25) gleich oder kleiner ist als der minimale Strömungsquerschnitt in dem Durchleitungsbereich.

23. System nach einem der Ansprüche 21 bis 22, **dadurch gekennzeichnet, dass** der Strömungsquerschnitt in dem Bereich zwischen der Abzweigung (29) und der Adsorptions- und/oder Absorptions- und/oder Filtereinrichtung (25) gegenüber dem minimalen Strömungsquerschnitt in dem Durchleitungsbereich durch ein Hindernis verengt ist, vorzugsweise wobei das Hindernis als eine Zunge (24a) ausgebildet ist, die innerhalb des Durchleitungsbereichs etwa auf Höhe der Abzweigung (29) angeordnet ist, und/oder wobei vorzugsweise das Hindernis als eine Zunge (24a) ausgebildet ist, die distal der Abzweigung (29) angelenkt ist und die Abzweigung (29) zu der Adsorptions- und/oder Absorptions- und/oder Filtereinrichtung (25) gegenüber dem Durchleitungsbereich im Falle der Durchleitung von Substanzen zumindest bereichsweise abdeckt, insbesondere wobei die Zunge (24a) biegsam und/oder elastisch ausgebildet ist, und/oder vorzugsweise aus Kunststoff besteht.

24. System nach einem der Ansprüche 21 bis 23, **dadurch gekennzeichnet, dass** die dem Durchleitungsbereich oder der Abzweigung (29) zugewandte Einlassöffnung der Adsorptions- und/oder Absorptions- und/oder Filtereinrichtung (25) durch eine gasdurchlässige, vorzugsweise aber wasserabweisende Trennlage (27) abgedeckt ist, vorzugsweise wobei die Trennlage (27) eine gering benetzbare Oberfläche aufweist (Lotoseffekt), insbesondere an ihrer dem Durchleitungsbereich oder der Abzweigung (29) zugewandten Seite, und/oder wobei vorzugsweise die Trennlage (27) aus einem Papier gebildet ist, inbesondere aus einem Vliespapier, oder als mikroporöse Membran ausgebildet ist, beispielsweise aus expandiertem Polytetrafluorethylen, bevorzugt mit einem maximalen Porendurchmesser von weniger als 1 mm, vorzugsweise mit einem maximalen Porendurchmesser von 100 $\mu$m oder weniger, insbesondere mit einem maximalen Porendurchmesser von 10 $\mu$m oder weniger.

25. System nach einem der Ansprüche 21 bis 24, **dadurch gekennzeichnet, dass** die Adsorptions- und/oder Absorptions- und/oder Filtereinrichtung (25) eine oder mehrere oberflächenaktive, die Geruchsstoffe auf der Oberfläche anlagernde, adsorbierenden Substanzen, aufweist, und/oder eine oder mehrere absorbierende, die geruchswirksamen Stoffe in Art einer physikalischer Lösung aufnehmende Substanzen.

26. System nach einem der Ansprüche 21 bis 25, **dadurch gekennzeichnet, dass** die Adsorptions- und/oder Absorptions- und/oder Filtereinrichtung (25) ein adsorbierendes oder absorbierendes oder filterndes Granulat umasst, beispielsweise Aktivkohle, beispielsweise in einer Menge von 100 ml oder weniger, bevorzugt in einer Menge von 50 ml oder weniger, insbesondere in einer Menge von 20 ml oder weniger, oder in einer Menge von 10 ml oder weniger, oder gar in einer Menge von 5 ml oder weniger, vorzugsweise wobei die Adsorptions- und/oder Absorptions- und/oder Filtereinrichtung (25)

a) das Granulat, beispielsweise die Aktivkohle, in einer Menge von 2 ml oder mehr enthält, bevorzugt in einer Menge von 5 ml oder mehr, insbesondere in einer Menge von 10 ml oder mehr, oder in einer Menge von 20 ml oder mehr, oder gar in einer Menge von 30 ml oder mehr, und/oder
b) in ihrem Inneren ein oder mehrere lamellenartige Strukturen aufweist, um das zu reinigende Gas durch die Adsorptions- und/oder Absorptions- und/oder Filtereinrichtung (25) zu führen, wobei vorzugsweisse mehrere lamellenartigen Strukturen gegeneinander versetzt sind und/oder nach Art von verschränkten Fingern ineinander greifen, und/oder
c) in ihrem Inneren wenigstens eine schnecken-, spiral- und/oder wendelförmige Struktur aufweist, um das zu reinigende Gas durch die Adsorptions- und/oder Absorptions- und/oder Filtereinrichtung (25) zu führen, und/oder
d) ein geruchsentfaltende Stoffe filterndes Material aufweist, insbesondere im Bereich ihrer stromabwärtigen bzw. auslassseitigen Öffnung (25a), und/oder
e) ein infektiöse Erreger filterndes Material aufweist, insbesondere im Bereich ihrer stromabwärtigen bzw. auslassseitigen Öffnung (25a).

27. System nach einem der Ansprüche 21 bis 26, **dadurch gekennzeichnet, dass** die Adsorptions- und/oder Absorptions- und/oder Filtereinrichtung (25) in einer auswechselbaren Kartusche (25a) oder in einer abnehmbaren Kappe angeordnet ist, vorzugsweise wobei die auswechselbare Kartusche (25a) oder die abnehmbaren Kappe im Bereich ihrer freien Stirnseite oder ihres Umfangs mit einer oder mehreren Auslassöffnungen versehen ist, und/oder wobei vorzugsweise die auswechselbare Kartusche (25a) oder die abnehmbaren Kappe über einen Bajonettverschluss oder über einen Schraubverschluss mit dem Durchleitungsbereich an der dortigen Abzweigung verbindbar ist, und/oder wobei vorzugsweise der die Abzweigung aufweisende Durchleitungsbereich und/oder die auswechselbare Kartusche (254) und/oder die abnehmbaren Kappe wenigstens ein Kompartiment zur Aufnahme eines Duftstoffs aufweist, und/oder wobei vorzugsweise der die Abzweigung (29) aufweisende Durchleitungsbereich (24) mit dem Schlauch (2) oder dem Beutel (11) integriert ist.

28. System nach einem der Ansprüche 21 bis 27, **dadurch gekennzeichnet, dass** der die Abzweigung (29) aufweisende Durchleitungsbereich (24) mit dem Katheter (5) integriert oder verbindbar ist, der über das Ballonelement (3) im Patienten verankerbar ist.

**Claims**

1. A closed system for the delivery and/or discharge of odor-intensive substances to/from the body of a patient in the most odor-neutral manner possible, comprising

- a head unit (5) that is positionable in an interior space of the body of the patient, includes a balloon-like element (3), and retains and/or seals the system in the body of the patient,
- an extracorporeal container (11) including a preferably film-based, baglike compartment for receiving the substances to be delivered and/or discharged, and
- a tubular film-like structure (2) that connects the body interior space that is to be reached to the extracorporeal container,

**characterized in that** the delivering and/or discharging, tubular film-like structure (2) of the system and the film-based components of the container (11) are made of multilayer film material which in each case includes at least one barrier layer (34), made of ethylene-vinyl alcohol copolymer (EVOH) and/or polyvinylidene chloride (PVDC), that acts as an odor barrier, and in each case at least one carrier layer (35, 37, 39, 49) made of a robust, mechanically loadable material, wherein the material of a carrier layer is selected from the group comprising polyurethane (PUR), preferably thermoplastic polyurethane (TPU), polyvinyl chloride (PVC), polyamide (PA), thermoplastic polyamide elastomer (TPE-A), and polyolefin-based polymers, as well as combinations of these materials, like, for example, a combination of one carrier layer made of PVC with at least one carrier layer made of PUR.

2. The system according to Claim 1, **characterized in that** the film-based tube components and/or balloon components of the head unit (5) positioned in the interior space of the body are also made of multilayer film material which in each case includes at least one barrier layer (34), made of ethylene-vinyl alcohol copolymer (EVOH) and/or polyvinylidene chloride (PVDC), that acts as an odor barrier, and in each case at least one carrier layer (35, 37, 39, 49) made of a robust, mechanically loadable material.

3. The system according to Claim 1 or 2, **characterized in that** the (overall) thickness $d_T$ of the carrier layer or of all carrier layers is greater than the (overall) thickness $d_B$ of the barrier layer or of all barrier layers:

$$d_T > d_B,$$

or that the (overall) thickness $d_T$ of the carrier layer or of all carrier layers is greater than or equal to five times the (overall) thickness $d_B$ of the barrier layer or of all barrier layers:

$$d_T \geq 5 * d_B,$$

or that the (overall) thickness $d_T$ of the carrier layer or of all carrier layers is greater than or equal to ten times the (overall) thickness $d_B$ of the barrier layer or of all barrier layers:

$$d_T \geq 10 * d_B,$$

or that the (overall) thickness $d_T$ of the carrier layer or of all carrier layers is greater than or equal to twenty times the (overall) thickness $d_B$ of the barrier layer made of EVOH or PVDC or of all barrier layers made of EVOH and/or PVDC:

$$d_T \geq 20 * d_B,$$

or that the (overall) thickness $d_T$ of the carrier layer or of all carrier layers is greater than or equal to forty times the (overall) thickness $d_B$ of the barrier layer made of EVOH or PVDC or of all barrier layers made of EVOH and/or PVDC:

$$d_T > 40 * d_B.$$

4. The system according to one of the preceding claims, **characterized in that** at least one carrier layer is made of a mechanically loadable, preferably elastically deformable and elastically straightening, carrier material.

5. The system according to one of the preceding claims, **characterized in that** the material is made up of at least one carrier layer made of PUR having a Shore hardness of 80A to 95A or 55D to 65D, and/or of a thermoplastic PUR of a type having a water absorption according to DIN ISO 62 of 5% or less, preferably having a water absorption according to DIN ISO 62 of 2% or less.

6. The system according to one of the preceding claims, **characterized in that** at least one barrier layer of the two- or multilayer film material is joined to a carrier layer only at one side, preferably wherein the barrier layer is made of PVDC, especially wherein at least one carrier layer is made of PVC or PUR.

7. The system according to one of Claims 1 through 6, **characterized in that** the multilayer film material is made up of a sandwich-like combination, at least one central barrier layer being enclosed on both sides by at least one carrier layer each, preferably wherein a carrier layer made of PVC is provided at one or both sides of the central barrier layer, and/or wherein preferably a carrier layer made of PUR is provided at one or both sides of the central barrier layer, and/or wherein especially at least one carrier layer made of PVC is combined with at least one carrier layer made of PUR at each side of a central barrier layer.

8. The system according to one of the preceding claims, **characterized in that** the wall thickness of the multilayer film material is in a range of 10 $\mu$m to 5 mm, and/or in a range of 20 $\mu$m to 2 mm, for example in a range of 50 $\mu$m to 1 mm, preferably in a range of 50 $\mu$m to 1 mm, in particular in a range of 100 $\mu$m to 500 $\mu$m.

9. The system according to one of the preceding claims, **characterized in that** the thickness of a barrier layer is in a range of 5 to 25 $\mu$m, preferably in a range of 10 to 15 $\mu$m.

10. The system according to one of the preceding claims, **characterized by** the following structure of the multilayer film material:

    - PVC, for example having a Shore hardness of 60A to 80A and a thickness of 140 to 280 $\mu$m, at the inner side facing a central lumen, as an inner carrier layer,
    - PUR, for example having a Shore hardness of 55D and a thickness of 50 to 100 $\mu$m, at the outer side facing away from the central lumen, as an outer carrier layer, and
    - EVOH or PVDC having a thickness of 10 to 20 $\mu$m between the inner carrier layer and the outer carrier layer, as a central barrier layer.

11. The system according to one of the preceding claims, **characterized in that** one or more layers of the multilayer film material are coextruded with one another.

12. The system according to one of the preceding claims, **characterized in that** at least one barrier layer, in particular a barrier layer made of EVOH, is joined to a neighboring layer made of PA, in particular without a connecting, adhesion-assisting tie layer, and/or **in that** at least one carrier layer, in particular a carrier layer made of PUR, is joined to a neighboring layer made of PA, in particular without a connecting, adhesion-assisting tie layer, and/or **in that** a layer made of PA is joined to a PE-based layer by use of an adhesion promoter as an intermediate layer.

13. The system according to one of the preceding claims, **characterized in that** different, separately manufactured, adjacently situated components in the system, such as the head unit and the tubular film-like structure on the one hand or the container and the tubular film-like structure on the other hand, are adhesively bonded to one another, for example by adhering adjacently situated surfaces of two separately manufactured components using a solvent, in particular using a solvent such as cyclohexanone or tetrahydrofuran.

14. The system according to one of the preceding claims, **characterized in that** the balloon and the line are made of the same multilayer film material, and/or the container and the line are made of the same multilayer film material, and/or the balloon, the container, and the line are made of the same multilayer film material, preferably wherein components that are made of the same multilayer film material are integrated with one another and/or manufactured as a unit.

15. The system according to one of the preceding claims, **characterized in that** the multilayer film material, at least in areas, is provided with a spiral-shaped indentation and/or protrusion or with multiple successive, ring-shaped, inwardly directed indentations and/or protrusions in order to assist the spontaneous straightening of the film material in question after a temporary deformation, preferably wherein circular expansions or reductions of the multilayer film material

    a) are 3 to 6 mm wide, and/or
    b) are 1.0 to 3 mm deep, preferably 1.5 to 2.5 mm deep, at the apex.

16. The system according to one of the preceding claims, **characterized**

    a) **in that** the catheter balloon has a radially expanded, intracorporeal balloon segment and a transosteal balloon segment that is radially tapered with respect to same, and/or
    b) **in that** the entire intracorporeal portion and also the entire extracorporeal portion of the catheter balloon are formed from a single, structurally continuous film tube blank.

17. The system according to one of the preceding claims, **characterized in that** multiple layers of the multilayer film material are thermally formed together in a subsequent manufacturing step, for example using a hot-molding process, an initially unprocessed tube blank being expanded into a heated mold cavity via action by compressed air, and its shape being fixed there via subsequent cooling of the mold.

18. The system according to one of the preceding claims, **characterized in that** the forming of an extracorporeal, stool-discharging tube segment to form a tube casing that is provided with lumen-stabilizing indentations and/or protrusions, for example, takes place simultaneously with the formation of an intracorporeal balloon component of the catheter balloon and/or of a transosteal balloon component of the catheter balloon in a single work step.

19. The system according to one of the preceding claims, **characterized in that** during the in situ placement of a residual balloon body i.e., a balloon body that is formed with excess balloon material along the balloon circumference, typical invaginated indentations (21) of the excess, residual balloon envelope form in the balloon interior, preferably wherein the indentations (21) that are invaginated in the balloon interior have turned-over formations with an eyelet-like cross section, which preferably extend or continue as channel-like formations in the longitudinal direction of the balloon (3), i.e., between the distal and proximal end-face sides of the balloon, especially wherein the turned-over formations with an eyelet-like cross section, which preferably extend or continue as channel-like formations in the longitudinal direction of the balloon (3), have an opening diameter between 30 $\mu$m and 120 $\mu$m, preferably an eyelet diameter between 40 $\mu$m and 80 $\mu$m, at a filling pressure of the balloon (3) of 30 mbar.

20. The system according to one of the preceding claims, **characterized in that** a gas- and/or water vapor-tight barrier layer (34) is situated between an elastically deformable carrier layer made of PUR and a nonelastically deformable carrier layer made of PVC, for example, preferably wherein the proportional wall thickness of the nonelastically deformable carrier layer (33, 35, 36, 39, 40) made of PVC, for example, is greater than the proportional wall thickness(es) of the elastically deformable carrier layer(s) made of PUR.

21. The system according to one of the preceding claims, **characterized by** a degassing device having an outlet at which an internal overpressure within the system may be reduced by the release of gas, and having an adsorption and/or absorption and/or filter device that filters undesirable substances, in particular odorous substances, from the released gas, preferably wherein the degassing device is connected to a compartment of the container that receives the particular delivering and/or discharging substance, and/or wherein a branch that is used as an outlet is provided in a conducting area for conducting the substances to be delivered and/or discharged, especially wherein the adsorption and/or absorption and/or filter device is integrated with the branch or connected to same, wherein, in particular, the adsorption and/or absorption and/or filter device is situated spatially separate from the delivering and/or discharging lumen of the system via a protected access path in the branch, so that the degassing unit having an adsorbent or filtering action is protected from direct exposure to the substances to be delivered and/or discharged, and/or wherein preferably the branch is situated distally with respect to a return-inhibiting element at the inlet of the compartment in the container, especially wherein the return-inhibiting element has film-based, lip-like, or sail-like components (31), the distance between the film-based, lip-like, or sail-like components (31) in the main flow direction, i.e., in the direction from the drainage line (2) to the collection compartment of the collection container (11), preferably being tapered.

22. The system according to Claim 21, **characterized in that** the adsorption and/or absorption and/or filter device (25) is spaced apart from the conducting area, at least in areas, to avoid, to the greatest extent possible, intensive contact with the substances flowing through the conducting area, and/or **in that** the longitudinal direction of the flow channel between the branch (29) and the adsorption and/or absorption and/or filter device, with respect to the longitudinal direction of the conducting area between the branch (29) and the drain connection, encloses an angle of 90° or less than 90°, for example an angle of 75° or less, preferably an angle of 60° or less, in particular an angle of 45° or less, or even an angle of 30° or less, and/or **in that** the maximum flow cross section in the area between the branch (29) and the adsorption and/or absorption and/or filter device (25) is equal to or less than the minimum flow cross section in the conducting area.

23. The system according to one of Claims 21 through 22, **characterized in that** the flow cross section in the area between the branch (29) and the adsorption and/or absorption and/or filter device (25) is narrowed with respect to the minimum flow cross section in the conducting area via an obstruction, preferably wherein the obstruction is designed as a tongue (24a) that is situated within the conducting area approximately at the height of the branch (29), and/or wherein preferably the obstruction is designed as a tongue (24a) that is articulated distally with respect to the branch (29), and that covers the branch (29), at least in areas, for the adsorption and/or absorption and/or filter device (25) with respect to the conducting area when substances are conducted, especially wherein the tongue (24a) has a flexible and/or elastic design, and/or is preferably made of plastic.

24. The system according to one of Claims 21 through 23, **characterized in that** the inlet opening of the adsorption and/or absorption and/or filter device (25) facing the conducting area or the branch (29) is covered by a gas-permeable, but preferably water-repellent, separating layer (27), preferably wherein the separating layer (27) has a slightly wettable surface (lotus effect), in particular on its side facing the conducting area or the branch (29), and/or wherein preferably the separating layer (27) is made of a paper, in particular a nonwoven paper, or is designed as a microporous membrane made of expanded polytetrafluoroethylene, for example, preferably having a maximum pore diameter of less than 1 mm, preferably having a maximum pore diameter of 100 $\mu$m or less, in particular having a maximum pore diameter of 10 $\mu$m or less.

25. The system according to one of Claims 21 through 24, **characterized in that** the adsorption and/or absorption and/or filter device (25) has one or more surface-active adsorbent substances that accumulate odorous substances on the surface, and/or one or more absorbent substances that absorb the odoreffective materials in the manner of a physical solution.

26. The system according to one of Claims 21 through 25, **characterized in that** the adsorption and/or absorption and/or filter device (25) includes an adsorbent or absorbent or filtering granulate, for example activated carbon, for example in a quantity of 100 mL or less, preferably in a quantity of 50 mL or less, in particular in a quantity of 20 mL or less, or in a quantity of 10 mL or less, or even in a quantity of 5 mL or less, preferably wherein the adsorption and/or absorption and/or filter device (25)

a) contains the granulate, for example the activated carbon, in a quantity of 2 mL or greater, preferably in a quantity of 5 mL or greater, in particular in a quantity of 10 mL or greater, or in a quantity of 20 mL or greater, or even in a quantity of 30 mL or greater, and/or
b) in its interior has one or more lamella-like structures in order to lead the gas to be cleaned through the adsorption and/or absorption and/or filter device (25), preferably multiple lamella-like structures being offset relative to one another and/or meshing into one another in the manner of interlocked fingers, and/or
c) in its interior has at least one screw-shaped, spiral, and/or helical structure in order to lead the gas to be cleaned through the adsorption and/or absorption and/or filter device (25), and/or
d) includes a material that filters odor-causing substances, in particular in the area of its downstream or outlet-side opening (25a), and/or
e) includes a material that filters infectious pathogens, in particular in the area of its downstream or outlet-side opening (25a).

27. The system according to one of Claims 21 through 26, **characterized in that** the adsorption and/or absorption and/or filter device (25) is situated in a replaceable cartridge (25a) or in a removable cap, preferably wherein the replaceable cartridge (25a) or the removable cap is provided with one or more outlet openings in the area of its free end-face side or its circumference, and/or wherein preferably the replaceable cartridge (25a) or the removable cap is connectable to the conducting area at the branch at that location via a bayonet lock or via a screw closure, and/or wherein preferably the conducting area including the branch, and/or the replaceable cartridge (25a) and/or the removable cap, has at least one compartment for accommodating a fragrance, and/or wherein preferably the conducting area (24) including the branch (29) is integrated with the tube (2) or the bag (11).

**28.** The system according to one of Claims 21 through 27, **characterized in that** the conducting area (24) including the branch (29) is integrated with or connectable to the catheter (5), which is anchorable in the patient via the balloon element (3).

## Revendications

**1.** Système fermé pour une administration et/ou une évacuation au maximum neutre en odeurs de substances odorantes dans/depuis le corps d'un patient, comportant

- une unité de tête (5) pouvant être positionnée dans un espace intérieur du corps du patient, présentant un élément de type ballonnet (3), retenant et/ou étanchéifiant le système dans le corps du patient,
- un récipient (11) disposé de manière extracorporelle avec un compartiment de préférence à base de film, en forme de poche pour la réception des substances à administrer et/ou à évacuer, ainsi qu'
- une structure de type film tubulaire (2) qui relie l'espace intérieur du corps à atteindre au récipient disposé de manière extracorporelle,

**caractérisé en ce que** la structure de type film tubulaire (2) d'administration et d'évacuation du système ainsi que les composants à base de film du récipient (11) sont constitués d'un matériau de film multicouche qui présente respectivement au moins une couche barrière (34) agissant comme barrière contre les odeurs en copolymère d'éthylène-alcool vinylique (EVOH) et/ou en chlorure de polyvinylidène (PVDC), ainsi que respectivement au moins une couche support (35, 37, 39, 40) en un matériau robuste, résistant aux contraintes mécaniques, **en ce que** le matériau d'une couche support est choisi parmi le groupe comprenant le polyuréthane (PUR), de préférence le polyuréthane thermoplastique (TPU), le chlorure de polyvinyle (PVC), le polyamide (PA), l'élastomère polyamide thermoplastique (TPE-A) et les polymères à base de polyoléfine ainsi que des combinaisons de ces matériaux comme par exemple une combinaison d'une couche support en PVC et d'au moins une couche support en PUR.

**2.** Système selon la revendication 1, **caractérisé en ce que** les composants de tube et/ou de ballonnet à base de film de l'unité de tête (5) positionnée dans l'espace intérieur du corps sont également constitués de matériau de film multicouche qui présente respectivement au moins une couche barrière (34) agissant comme barrière contre les odeurs en copolymère d'éthylène-alcool vinylique (EVOH) et/ou en chlorure de polyvinylidène (PVDC), ainsi que respectivement au moins une couche support (35, 37, 39, 40) en un matériau robuste, résistant aux contraintes mécaniques.

**3.** Système selon la revendication 1 ou 2, **caractérisé en ce que** l'épaisseur (totale) $d_T$ de la couche support ou de toutes les couches support est supérieure à l'épaisseur (totale) $d_B$ de la couche barrière ou de toutes les couches barrières :

$$d_T > d_B,$$

ou **en ce que** l'épaisseur (totale) $d_T$ de la couche support ou de toutes les couches supports est égale ou supérieure à cinq fois l'épaisseur (totale) $d_B$ de la couche barrière ou de toutes les couches barrières :

$$d_T \geq 5 * d_B,$$

ou **en ce que** l'épaisseur (totale) $d_T$ de la couche support ou de toutes les couches supports est égale ou supérieure à dix fois l'épaisseur (totale) $d_B$ de la couche barrière ou de toutes les couches barrières :

$$d_T \geq 10 * d_B,$$

ou **en ce que** l'épaisseur (totale) $d_T$ de la couche support ou de toutes les couches supports est égale ou supérieure à vingt fois l'épaisseur (totale) $d_B$ de la couche barrière en EVOH ou en PVDC ou de toutes les couches barrières en EVOH et/ou en PVDC :

$$d_T \geq 20 \; {}^*d_{B,}$$

ou **en ce que** l'épaisseur (totale) $d_T$ de la couche support ou de toutes les couches supports est égale ou supérieure à quarante fois l'épaisseur (totale) $d_B$ de la couche barrière en EVOH ou en PVDC ou de toutes les couches barrières en EVOH et/ou en PVDC :

$$d_T \geq 40 \; {}^*d_B.$$

4. Système selon l'une des revendications précédentes, **caractérisé en ce qu'**au moins une couche support est constituée d'un matériau support résistant aux contraintes mécaniques, de préférence pouvant se déformer élastiquement et pouvant se redresser élastiquement.

5. Système selon l'une des revendications précédentes, **caractérisé en ce que** le matériau d'au moins une couche support est constitué de PUR présentant une dureté Shore de 80A à 95A ou de 55D à 65D, et/ou est constitué d'un PUR thermoplastique d'un type ayant une absorption d'eau selon DIN ISO 62 de 5 % ou moins, de préférence ayant une absorption d'eau selon DIN ISO 62 de 2 % ou moins.

6. Système selon l'une des revendications précédentes, **caractérisé en ce qu'**au moins une couche barrière du matériau de film bicouche ou multicouche est reliée à une couche support uniquement sur un côté, de préférence **en ce que** la couche barrière est constituée de PVDC, en particulier **en ce qu'**au moins une couche support est constituée de PVC ou de PUR.

7. Système selon l'une des revendications 1 à 6, **caractérisé en ce que** le matériau de film multicouche est constitué d'une combinaison de type sandwich, **en ce qu'**au moins une couche barrière centrale est entourée des deux côtés par au moins une couche support, de préférence **en ce que** sur l'un ou les deux côtés de la couche barrière centrale est prévue une couche support en PVC, et/ou **en ce que** de préférence d'un côté ou des deux côtés de la couche barrière centrale est prévue une couche support en PUR, et/ou **en ce qu'**en particulier de chaque côté d'une couche barrière centrale au moins une couche support en PVC est combinée avec au moins une couche support en PUR.

8. Système selon l'une des revendications précédentes, **caractérisé en ce que** l'épaisseur de paroi du matériau de film multicouche est dans une plage comprise entre 10 $\mu$m et 5 mm, et/ou dans une plage entre 20 $\mu$m et 2 mm, par exemple dans une plage entre 50 $\mu$m et 1 mm, de préférence dans une plage entre 50 $\mu$m et 1 mm, en particulier dans une plage entre 100 $\mu$m et 500 $\mu$m.

9. Système selon l'une des revendications précédentes, **caractérisé en ce que** l'épaisseur d'une couche barrière est comprise dans une plage entre 5 et 25 $\mu$m, de préférence dans une plage entre 10 et 15 $\mu$m.

10. Système selon l'une des revendications précédentes, **caractérisé par** la structure suivante du matériau de film multicouche :

    - PVC, par exemple d'une dureté Shore de 60A à 80A et d'une épa épaisseur de 140 à 280 $\mu$m sur la face interne tournée vers une lumière centrale comme couche support intérieure
    - PUR, par exemple d'une dureté Shore de 55D et d'une épaisseur de 50 à 100 $\mu$m sur la face externe opposée à la lumière centrale comme couche support extérieure, ainsi que
    - EVOH ou PVDC d'une épaisseur de 10 à 20 $\mu$m entre la couche support intérieure et la couche support extérieure comme couche barrière centrale.

11. Système selon l'une des revendications précédentes, **caractérisé en ce qu'**une ou plusieurs couches du matériau de film multicouche sont coextrudées les unes avec les autres.

12. Système selon l'une des revendications précédentes, **caractérisé en ce qu'**au moins une couche barrière, en particulier une couche barrière en EVOH, est reliée à une couche adjacente en PA, en particulier sans couche de liaison liante, favorisant l'adhésion, et/ou **en ce qu'**au moins une couche support, en particulier une couche support en PUR est reliée à une couche adjacente en PA, en particulier sans couche de liaison liante, favorisant l'adhésion, et/ou **en ce qu'**une couche en PA est reliée à une couche à base de PE par l'intermédiaire d'un agent adhésif comme couche intermédiaire.

13. Système selon l'une des revendications précédentes, **caractérisé en ce que** différents composants adjacents dans le système, fabriqués séparément, tels que l'unité de tête et la structure de type film tubulaire d'une part ou le récipient et la structure de type film tubulaire d'autre part, sont collés les uns aux autres, par exemple **en ce que** des surfaces adjacentes de deux composants fabriqués séparément sont collés avec un solvant, en particulier avec un solvant comme la cyclohexanone ou le tétrahydrofurane.

14. Système selon l'une des revendications précédentes, **caractérisé en ce que** le ballonnet et le conduit sont constitués du même matériau de film multicouche, et/ou **en ce que** le récipient et le conduit sont constitués du même matériau de film multicouche, et/ou **en ce que** le ballonnet, le récipient et le conduit sont constitués du même matériau de film multicouche, de préférence **en ce que** des composants, qui sont constitués du même matériau de film multicouche, sont intégrés entre eux et/ou sont fabriqués en tant qu'unité.

15. Système selon l'une des revendications précédentes, **caractérisé en ce que** le matériau de film multicouche est pourvu au moins partiellement d'un retournement et/ou d'une protubérance en forme de spirale ou de plusieurs retournements et/ou protubérances orientés vers l'intérieur ou l'extérieur, en série, de forme annulaire afin de favoriser le redressement spontané du matériau de film concerné après une déformation passagère, de préférence **en ce que** des extensions circulaires ou réductions du matériau de film multicouche

    a) ont une largeur de 3 à 6 mm, et/ou
    b) ont une profondeur au sommet de 1,0 à 3 mm, de préférence de 1,5 à 2,5 mm.

16. Système selon l'une des revendications précédentes, **caractérisé**

    a) **en ce que** le ballonnet de cathéter comporte un segment de ballonnet intracorporel, radialement élargi ainsi qu'un segment de ballonnet transostial radialement rétréci par rapport à celui-ci, et/ou
    b) **en ce qu'**aussi bien l'ensemble de la partie intracorporelle que l'ensemble de la partie extracorporelle du ballonnet de cathéter sont formés à partir d'une seule ébauche de tube de film structurellement continue.

17. Système selon l'une des revendications précédentes, **caractérisé en ce que** plusieurs couches du matériau de film multicouche sont thermoformées ensemble lors d'une étape de fabrication ultérieure, par exemple selon un procédé de moulage à chaud, **en ce qu'**une ébauche de tube d'abord non traitée est expansée dans une cavité de moulage chauffée par alimentation en air comprimé, et y fixée dans son moule par refroidissement ultérieur de l'outil de formage.

18. Système selon l'une des revendications précédentes, **caractérisé en ce que**, simultanément au reformage d'un segment de tube extracorporel pour l'évacuation de selles par exemple en une gaine de tube pourvue de retournements et/ou de protubérances stabilisant la lumière, est effectué également le formage d'un composant de ballonnet intracorporel du ballonnet de cathéter et/ou d'un composant de ballonnet transostial du ballonnet de cathéter en une seule opération.

19. Système selon l'une des revendications précédentes, **caractérisé en ce que**, lors du placement in situ d'un corps de ballonnet résiduel, c'est-à-dire formé avec un matériau de ballonnet excédentaire le long de la circonférence du ballonnet, il se forme des retournements (21) typiques invaginés à l'intérieur du ballonnet de l'enveloppe de ballonnet résiduelle excédentaire, de préférence **en ce que** les retournements (21) invaginés à l'intérieur du ballonnet présentent des formations de repliement en forme d'oeillet en section transversale, qui s'étendent ou se prolongent de préférence dans le sens longitudinal du ballonnet (3), c'est-à-dire entre sa face frontale distale et sa face frontale proximale, en tant que formations de type canal, en particulier **en ce que** les formations de repliement en forme d'oeillet en section transversale, qui s'étendent ou se prolongent de préférence dans le sens longitudinal du ballonnet (3) en tant que formations de type canal, présentent à une pression de remplissage du ballonnet (3) de 30 mbar un diamètre d'ouverture compris entre 30 $\mu$m et 120 $\mu$m, de préférence un diamètre d'oeillet compris entre 40 $\mu$m et 80 $\mu$m.

20. Système selon l'une des revendications précédentes, **caractérisé en ce qu'**entre une couche support élastiquement déformable en PUR et une couche support non élastiquement déformable, par exemple en PVC, est disposée une couche barrière (34) étanche au gaz et/ou à la vapeur d'eau, de préférence **en ce que** l'épaisseur de paroi proportionnelle de la couche support (33, 35, 36, 39, 40) non élastiquement déformable, par exemple en PVC, est supérieure à l'épaisseur de paroi/ aux épaisseurs de paroi proportionnelle(s) de la/des couches support(s) élastiquement déformable(s) en PUR.

21. Système selon l'une des revendications précédentes, **caractérisé par** un dispositif de dégazage comportant une sortie au niveau de laquelle une surpression interne à l'intérieur du système peut être réduite par la sortie de gaz, et comportant un dispositif d'adsorption et/ou d'absorption et/ou de filtration qui filtre les substances indésirables du gaz sortant, en particulier des substances odorantes, de préférence en ce que le dispositif de dégazage est raccordé à un compartiment recevant la substance respective qui doit être administrée et/ou évacuée du récipient, et/ou en ce qu'une dérivation est prévue de préférence dans une zone de passage pour le passage des substances qui doivent être administrées et/ou évacuées, ladite dérivation servant de sortie, en particulier en ce que le dispositif d'adsorption et/ou d'absorption et/ou de filtration est intégré à la dérivation ou est raccordé à celle-ci, en particulier en ce que le dispositif d'adsorption et/ou d'absorption et/ou de filtration est spatialement éloigné de la lumière à administrer et/ou à évacuer du système par l'intermédiaire d'une voie d'accès protégée dans la dérivation, de sorte que l'unité de dégazage à effet d'adsorption et/ou de filtration est protégée d'une exposition directe aux substances qui doivent être administrées et/ou évacuées, et/ou de préférence en ce que la dérivation est disposée de manière distale par rapport à un élément anti-retour à l'entrée du compartiment dans le récipient, en particulier en ce que l'élément anti-retour présente des composants à base de film, de type lèvre ou voile (31), de préférence en ce que la distance entre les composants à base de film, de type lèvre ou voile (31) dans le sens d'écoulement principal, donc dans le sens du conduit de drainage (2) vers le compartiment collecteur du récipient (11) collecteur, diminue.

22. Système selon la revendication 21, **caractérisé en ce que** le dispositif d'adsorption et/ou d'absorption et/ou de filtration (25) est espacée au moins partiellement de la zone de passage afin d'éviter autant que possible un contact intensif avec les substances traversant la zone de passage, et/ou **en ce que** le sens longitudinal du canal d'écoulement entre la dérivation (29) et le dispositif d'adsorption et/ou d'absorption et/ou de filtration avec le sens longitudinal de la zone de passage entre la dérivation (29) et le raccord d'évacuation forme un angle de 90° ou inférieur à 90°, par exemple un angle de 75° ou moins, de préférence un angle de 60° ou moins, en particulier un angle de 45° ou moins, voire un angle de 30° ou moins, et/ou **en ce que** la section transversale d'écoulement maximale dans la zone entre la dérivation (29) et le dispositif d'adsorption et/ou d'absorption et/ou de filtration (25) est égale ou inférieure à la section transversale d'écoulement minimale dans la zone de passage.

23. Système selon l'une des revendications 21 à 22, **caractérisé en ce que** la section transversale d'écoulement dans la zone entre la dérivation (29) et le dispositif d'adsorption et/ou d'absorption et/ou de filtration (25) par rapport à la section transversale d'écoulement minimale est rétrécie dans la zone de passage par un obstacle, de préférence **en ce que** l'obstacle est réalisé sous forme de languette (24a), qui est disposée à l'intérieur de la zone de passage approximativement à hauteur de la dérivation (29), et/ou de préférence **en ce que** l'obstacle est réalisé sous forme de languette (24a) qui est articulée de manière distale par rapport à la dérivation (29) et qui recouvre au moins partiellement la dérivation (29) vers le dispositif d'adsorption et/ou d'absorption et/ou de filtration (25) par rapport à la zone de passage en cas de passage de substances, en particulier **en ce que** la languette (24a) est réalisée de manière flexible et/ou élastique, et/ou est de préférence constituée de matière plastique.

24. Système selon l'une des revendications 21 à 23, **caractérisé en ce que** l'orifice d'entrée orienté vers la zone de passage ou la dérivation (29) du dispositif d'adsorption et/ou d'absorption et/ou de filtration (25) est recouvert par une couche de séparation (27) perméable au gaz, mais de préférence hydrofuge, de préférence **en ce que** la couche de séparation (27) présente une surface faiblement mouillable (effet lotus), en particulier au niveau de sa face orientée vers la zone de passage ou vers la dérivation (29), et/ou de préférence **en ce que** la couche de séparation (27) est formée à partir d'un papier, en particulier à partir d'un papier non tissé, ou est réalisée sous forme de membrane microporeuse, par exemple en polytétrafluoroéthylène expansé, de préférence d'un diamètre de pore maximal inférieur à 1 mm, de préférence d'un diamètre de pore maximal de 100 $\mu$m ou moins, en particulier d'un diamètre de pore maximal de 10 $\mu$m ou moins.

25. Système selon l'une des revendications 21 à 24, **caractérisé en ce que** le dispositif d'adsorption et/ou d'absorption et/ou de filtration (25) présente une ou plusieurs substances tensioactives, absorbantes, fixant les substances odorantes sur la surface, et/ou une ou plusieurs substances absorbantes, absorbant les substances odorantes à la manière d'une solution physique.

26. Système selon l'une des revendications 21 à 25, **caractérisé en ce que** dispositif d'adsorption et/ou d'absorption et/ou de filtration (25) comporte un granulé absorbant ou absorbant ou filtrant, par exemple du charbon actif, par exemple en une quantité égale à 100 ml ou moins, de préférence en une quantité de 50 ml ou moins, en particulier en une quantité de 20 ml ou moins, ou en une quantité de 10 ml ou moins, voire en une quantité de 5 ml ou moins, de préférence **en ce que** le dispositif d'adsorption et/ou d'absorption et/ou de filtration (25)

a) contient le granulé, par exemple le charbon actif, en une quantité de 2 ml ou plus, de préférence en une quantité de 5 ml ou plus, en particulier en une quantité de 10 ml ou plus, ou en une quantité de 20 ml ou plus, voire en une quantité de 30 ml ou plus, et/ou
b) comporte à l'intérieur une ou plusieurs structures en forme de lamelles afin de faire passer le gaz à purifier à travers le dispositif d'adsorption et/ou d'absorption et/ou de filtration (25), de préférence en ce que plusieurs structures en forme de lamelles sont décalées les unes par rapport aux autres et/ou s'engagent les unes dans les autres comme des doigts entrelacés, et/ou
c) comporte à l'intérieur une structure en forme de vis sans fin, de spirale et/ou hélicoïdale pour faire passer le gaz à purifier à travers le le dispositif d'adsorption et/ou d'absorption et/ou de filtration (25), et/ou
d) comporte un matériau filtrant des substances dégageant des odeurs, en particulier dans la zone de son ouverture (25a) en aval et/ou côté sortie, et/ou
e) comporte un matériau filtrant des agents infectieux, en particulier dans la zone de son ouverture (25a) en aval et/ou côté sortie.

27. Système selon l'une des revendications 21 à 26, **caractérisé en ce que** le dispositif d'adsorption et/ou d'absorption et/ou de filtration (25) est disposé dans une cartouche interchangeable (25a) ou dans un capuchon amovible, de préférence **en ce que** la cartouche interchangeable (25a) ou le capuchon amovible est muni dans la zone de sa face frontale libre ou de sa périphérie d'une ou de plusieurs ouvertures de sortie, et/ou de préférence **en ce que** la cartouche interchangeable (25a) ou le capuchon amovible peut être relié à la zone de passage au niveau de la dérivation qui s'y trouve par une fermeture à baïonnette ou une fermeture à vis, et/ou de préférence **en ce que** la zone de passage comportant la dérivation et/ou la cartouche interchangeable (25a) et/ou le capuchon amovible comporte au moins un compartiment destiné à recevoir une substance odorante, et/ou de préférence **en ce que** la zone de passage (24) comportant la dérivation (29) est intégrée au tube (2) ou à la poche (11).

28. Système selon l'une des revendications 21 à 27, **caractérisé en ce que** la zone de passage (24) comportant la dérivation (29) est intégrée ou peut être reliée au cathéter (5) qui peut être ancré dans le patient par l'intermédiaire de l'élément de ballonnet (3).

Fig. 1

Fig. 2

Fig.3

Fig. 4

Fig.5

Fig.6

5$^{(4)}$

10$^{(4)}$    6$^{(4)}$

Fig.7

5$^{(5)}$

17$^{(5)}$

6$^{(5)}$

Fig.8

13a(6)

5(6)

15(6)

14(6)

13(6)

Fig.9

5(7)

I

15(7)

13(7)   13a(7)

Fig.10

5(8)

XI

13(8)

14(8)

15(8)

XI

13a(8)

Fig.11

17(8)   XI

13(8)

XI

13a(8)

Fig.12

Fig.13

Fig.14

Fig.15

Fig.16

2

23a

12

26a

26

27

25

24

26a

26b

23b

22

Fig.17

23a

25

24b

27

24a

29

24

N

24c

23b

Fig.18

Fig.19

~~~~ 33
~~~~ 34
~~~~ 35

Fig.20

~~~~ 36
~~~~ 34
~~~~ 36

Fig.21

~~~~ 37
~~~~ 34
~~~~ 37

Fig.22

~~~~ 40
~~~~ 39
~~~~ 38
~~~~ 37
~~~~ 34
~~~~ 37
~~~~ 38
~~~~ 39
~~~~ 40

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2011142928 A1
- WO 2011138731 A2
- US 20150282978 A1
- WO 2013056013 A1
- US 20110125114 A1
- EP 0625343 A2
- EP 1514528 A2
- WO 2008052018 A2